# EUROPEAN PATENT APPLICATION

(11) **EP 2 416 267 A1**
(43) Date of publication of application: **08.02.2012**
(21) Application number: 10172090.2
(22) Date of filing: 05.08.2010
(51) Int. Cl.: G06F 19/00

(54) **Method of aggregating task data objects and for providing an aggregated view**

(71) Applicant: F. Hoffmann-La Roche AG, 4070 Basel (CH); Roche Diagnostics GmbH, 68305 Mannheim (DE)
(72) Inventor: Von Allmen, Bernhard, 5707 Seengen (CH); Steimle, Anton, 8630 Rüti (CH); Suter, Urs, 8049 Zürich (CH)
(74) Representative: Richardt Patentanwälte

(57) **Abstract**

The invention relates to a method being implemented by an analysis system (100), the method comprising the steps of:
A) receiving (201) task data objects,
B) grouping (202) the received task data objects into task data object groups,
C) for at least some of the task data object groups, aggregating (203) all task data objects contained in a task data object group to provide aggregated task data object groups, each aggregated task data object group comprising an aggregated data value, said aggregated data value being calculated from all task data objects being contained in said aggregated task data object group,
D) specifying (204) a GUI element (214-220) for each of the aggregated task data object groups, whereby the aggregated data value calculated for said aggregated task data object group is used for specifying said GUI element, said specified GUI element being representative of said aggregated task data object group, and
E) displaying (205) the specified GUI elements on a graphical user interface (122.1, 122.2, 130, 200) for providing an aggregated view.

## Description

### Field of the invention

The present invention relates to an analysis system and method for aggregating task data objects and providing an aggregated view, wherein at least some of the aggregated task data objects are indicative of a laboratory procedure.

### Background and related art

In analytical laboratories, in particular clinical laboratories, a multitude of analyses on biological samples are executed in order to determine physiological and/or biochemical parameters being indicative of a disease, nutrition habits, drug effectiveness and/or organ function.

With the advent of new high-throughput technologies and advancement in lab automation systems, the number and complexity of tasks performed in parallel in a laboratory by a multitude of different lab-devices has tremendously increased. While lab atomization helped to improve the quality of analysis results and to reduce payroll costs, lab automation has not rendered human interaction with automated or semi-automated laboratory workcell systems unnecessary. As lab automation allows performing a multitude of pre-analytical, analytical and/or post-analytical laboratory procedures in parallel by a multitude of different laboratory devices and/or laboratory workcell systems, a growing number of laboratory procedures executed in parallel has to be monitored and controlled.

Lab-devices may fail or run out of reagents or other consumables. Often, a human is required to refill an empty reagent box, to replenish a consumable or to repair a failed lab-device. In addition, in many cases human interpretation of the gathered measurement values is still required. Although fewer lab workers are required to execute diagnostic or analytical tasks, a large number of highly diverse tasks related to monitoring and operating an analysis system and related to evaluating the gathered result still require human intervention.

Prior art laboratory workflow management systems allow the assignment of tasks to be performed in the context of a laboratory workflow to a particular user, thereby helping to clarify responsibilities in monitoring, controlling and/or executing tasks. US 2009/0094529 A1 describes a clinical application system facilitating completion of clinical workflow tasks. The system allows associating tasks in a clinical workflow with external actors and includes a user interface displaying clinical information to a user. The tasks are represented as links in the user interface. Each of the links triggers access to a corresponding external actor.

US 2009/0099871 A1 describes a multi-screen healthcare information management system which allows a user to simultaneously access, display and manipulate various healthcare information that are logically related on multiple screens without jumping around or entering and exiting various screens. The system allows a physician to write orders while viewing other biomedically relevant data.

US 2003/0045958 A1 describes a method for providing a displayable schedule of tasks. In an embodiment, a list of tasks to be performed by a worker is compiled in response to receiving identification information of a worker and based on a list of service tasks.

### Summary of invention

The invention provides for an analysis system for analyzing biological samples, a method and a computer program product as claimed in the respective independent claims. Embodiments of the invention are given in the dependent claims.

Embodiments of the invention to provide an improved analysis system, an improved computer-implemented method and an improved computer program product for displaying tasks which need to be performed in order to execute or complete a laboratory workflow.

In a further aspect, embodiments of the invention aim to provide an improved analysis system, an improved computer-implemented method and an improved computer program product for assisting a worker in efficiently executing tasks having been assigned to him or her.

In a further aspect, embodiments of the invention aim to provide an improved analysis system, an improved computer-implemented method and an improved computer program product for assisting a worker in efficiently identifying and/or triggering actions which need to be performed in order to keep one or more monitored lab-devices operative.

This object is solved by the features of the independent claims. Preferred embodiments of the invention are given in the dependent claims.

The term '**lab-device**' as used herein encompasses any pre-analytical, analytical and/or post-analytical laboratory device which is operable to perform a laboratory workflow step on one or more biological samples. A lab-device can be, for example, a centrifuge, a capping- or decapping unit, a sample storage unit, a conveyor belt, an analyzer, an aliquoter, a photometer or the like. A lab-device can be an analyzer or a receptacle of a sample, an analytical adjuvant material, a reagent, a wash buffer, an auxiliary liquid, a pipette, pipette tip or bulb or the like. A lab-device can also be any decapper device, a sample preparation and distribution system, a post-analytical device, in particular an automated sample storage device, and the like.

The term "**analysis system**" as used herein encompasses any system of lab-devices which are operated and/or monitored collectively. Typically, but not necessarily so, an analysis system comprises one or more analyzers, but the term 'analysis system' may also refer to a system comprising only one or more pre-analytical and/or post-analytical devices. For the sake of simplicity, said systems of lab-devices are herein also referred to as 'analysis systems'.

An '**analyzer**' is a laboratory apparatus that can induce a reaction of a biological sample with a reagent for obtaining a measurement value. For example, an analyzer can measure light absorption, fluorescence, electrical potential or other physical or chemical characteristics of the reaction to provide the measurement data.

The term '**mobile device**' as used herein encompasses any mobile electronic appliance having an interface for communicating with a server computer, in particular any handheld battery powered mobile appliance, such as a mobile phone, a smart phone, a personal digital assistant (PDA) or another electronic appliance having a wireless interface for establishing a communication link with a server computer such as over a wireless digital cellular telecommunications network or another wireless communication channel.

The term '**drill-down analysis**' as used herein refers to the execution of data processing and data evaluation techniques including, for example, arithmetic and/or statistical calculations on a set of data objects, wherein the execution of said drill-down analysis is triggered by a user navigating among levels of data ranging from the most aggregated (up) to the most detailed (down).

The term '**data aggregation**' as used herein is any process in which information is gathered and expressed in a summary form. Data aggregation allows the gathering of information about particular data objects having been grouped together based on specific attributes.

A '**task**' as used herein encompasses any laboratory procedure to be performed on one or more biological samples. In addition, the term encompasses any operation to be performed on data being received from one or more lab-devices of an analysis system upon processing a biological sample. The term further encompasses any processing step to be executed by a worker on a lab-device in order to preserve or restore the operability of a lab-device. Accordingly, executing a task can comprise executing a pre-analytical, analytical or post-analytical workflow or workflow step by a lab-device, e.g. an analyzer or a centrifuge. Executing a task can also comprise evaluating measurement values obtained on one or more samples by a user or refilling an empty reagent container of a lab-device by said user.

The term '**task data object**' as used herein encompasses any data object being indicative of a task. In particular, the term refers to data objects being indicative of a laboratory procedure to be performed on one or more biological samples using one or more lab-devices. A task data object can be, for example, an instance of a class data object specified in an object oriented programming language such as Java or C#.

In accordance with embodiments of the invention a method being implemented by an analysis system is provided, the analysis system being used for processing biological samples, the method comprising the steps of:
A) receiving task data objects, each task data object comprising at least one attribute, wherein at least some of the task data objects are indicative of a laboratory procedure to be performed on at least one biological sample using at least one lab-device, the at least one lab-device belonging to the analysis system,
B) grouping the received task data objects into task data object groups, wherein all task data objects belonging to the same task data object group share the same attribute value or attribute value range of the at least one attribute,
C) for at least some of the task data object groups, aggregating (203) all task data objects contained in a task data object group to provide aggregated task data object groups, each aggregated task data object group comprising an aggregated data value, said aggregated data value being calculated from all task data objects being contained in said aggregated task data object group,
D) specifying a GUI element for each of the aggregated task data object groups, whereby the aggregated data value calculated for said aggregated task data object group is used for specifying said GUI element, said specified GUI element being representative of said aggregated task data object group, and
E) displaying the specified GUI elements on a graphical user interface for providing an aggregated view.

The expression 'aggregated data value being calculated from all task data objects' encompasses gathering data contained in said task data objects, processing said data and returning a result value, said result value being a summary form of the processed data. Such a calculation can comprise, for example, an arithmetic operation such as a user defined function, the calculation of a minimum or maximum value, the calculation of a sum or the like. All task data objects contained in a group of task data objects to be aggregated according to step C are contained in the resulting aggregated group of task data objects, wherein the aggregated task data object group in addition comprises the aggregated data value.

In accordance with embodiments of the invention, task data objects are received by a server computer. Depending on the embodiment, the task data objects may be received from a single or a plurality of sources. According to some embodiments, a relational database having stored therein one or more task data objects is used as source. Said database may be stored on a storage medium of the server computer or may be stored on a remote database server being accessible by the server computer via a network, e.g. the intranet of a laboratory. The database may also be implemented as a multitude of different databases hosted on one or more different computers. According to embodiments of the invention, the database may also be an integral part of a lab-device belonging to the analysis system.

Depending on the embodiment of the invention, the server computer can be a server or any other kind of standalone processing device being connected to at least one lab-device of the analysis system. According to some embodiments of the invention, the server computer is an integral component of a lab-device of the analysis system. According to still further embodiments, the functionality provided by the server computer can be accessed by other computers, in particular end-user computers. Access is provided e.g. in via the middleware or via the LIS of a laboratory.

According to embodiments of the invention, at least some of the task data objects are received dynamically from a lab-device of an analysis system, the lab-device being connected to the server computer via a network, e.g. an Intranet of a laboratory.

According to embodiments of the invention, at least some of the task data objects represent a test request and are received from a lab-device or from a further computer, said lab-device or said further computer providing a user with means to specify a test request. A test request can be, for example, a request to perform a particular analysis, e.g. an analysis determining the blood glucose concentration of a sample of a particular patient. The test request is represented as task data object and transmitted to the server computer. The further computer may be, for example, a computer of a physician, said computer of the physician being connected to the server computer via a network, e.g. the intranet of the laboratory or the Internet.

According to embodiments of the invention, at least some of the task data objects are received dynamically from a lab-device and represent tasks which have to be performed on said lab-device in order to restore or preserve its operability. For example, an analyzer requiring a particular reagent for executing an analysis which has run out of reagent submits a request to refill the container of said reagent to the server computer. Said request can be submitted in the form of a task data object to the server computer. Likewise, the server computer may comprise means for transforming the information contained in said request into a task data object.

According to further embodiments of the invention, the task data objects received by the server computer from a lab-device are indicative of an error state of said lab-device and/or are indicative of the task required to restore operability of said device. According to further embodiments, the task data objects received by the server computer from the one or more lab-devices comprise additional information on how the indicated task is to be executed by a user. A task data object being indicative of the task to refill a particular reagent for a particular lab-device may comprise, for example, data on the position of the lab-device, e.g. the room number and/or an identifier of the laboratory the lab-device belongs to. Said task data object may in addition or alternatively comprise data on the lot number of the reagent, on the storage room where a new bottle of reagent can be retrieved, on the shelf-life of the reagent etc.

Depending on the embodiment of the invention, receiving one or more task data objects can be executed via a push- or pull method or a combination thereof.

According to embodiments, the server computer retrieves task data objects stored in the relational database via the pull method on a regular basis. In addition, task data objects dynamically generated by a user in the form of e.g. a test request or dynamically generated by a lab-device e.g. in the form of an error or status message may be submitted from the lab-device to the server computer and/or the relational database via the push method.

Program modules managing the data exchange between server computer, the one or more lab-devices and the one or more sources for receiving the task data objects are, according to embodiment of the invention, integrated into the middleware of a laboratory or are integrated into a laboratory information system (LIS).

At least some of the received task data objects are indicative of a laboratory procedure to be performed on at least one biological sample using at least one lab-device. A laboratory procedure can be any pre-analytical, analytical or post analytical step of a laboratory workflow, including multiple steps of a workflow, to be executed on one or more biological samples. A biological sample can be, for example, a blood or serum sample of a patient. A biological sample may also be a multi-well plate, tissue slides, cell cultures, Chip assays or the like.

Each of the received task data objects comprises one or more attributes. An attribute can be, for example and without limitation:
- the type of the indicated task (e.g. lab-device maintenance, requested analytical tests, evaluation of measurement results);
- an urgency level of the indicated task; an urgency level indicates the urgency and priority of a task. Highly urgent tasks require immediate action of a user in order to preserve operability of the analysis system.
- the time at which the indicated task is to be executed, e.g. a particular date or time;
- a device-ID being indicative of a lab-device, said lab-device to be used for performing the indicated task,
- a component of a lab-device assigned to the indicated task, e.g. a calibrating unit being integral part of an analyzer,
- a user-ID of a user assigned to the indicated task,
- a role-ID of a user role assigned to the indicated task,
- a specification of a place the lab-device is located at, the lab-device to perform the indicated task; the specification can be, for example, a room number and/or an identifier of a laboratory,
- a specification of a place a biological sample is stored in or can be retrieved from, the biological sample to be processed in the indicated task,
- a pre-analytical, analytical or post-analytical procedure to be performed according to the indicated task, e.g. the determination of the glucose level in a blood sample,
- a type of a reagent used for executing the indicated task,
- a supplier ID of the supplier of a reagent used for executing the indicated task,
- a storage-room-ID and/or a storage device-DI of the room or device wherein a reagent used for executing the indicated task is stored, and
- a patient-ID of the patient providing the biological sample to be processed during the indicated task.

The received task data objects are grouped into task data object groups. All task data objects belonging to the same task data object group share the same value or value range of at least one attribute. For example, if the attribute used for performing the grouping step is a user-ID assigned to each of the received task data objects, if three different user-IDs have been specified and assigned to three laboratory workers, if each user-ID is assigned to at least one task data object and if each task data object has assigned exactly one user-ID, a grouping step based on the attribute 'user-ID' will lead to the formation of three groups of task data objects. According to further embodiments, a task data object can have assigned more than one user-ID and/or role-ID. Depending on the embodiment of the invention and on the attribute(s) used for grouping, the created groups can be disjoint or overlapping.

According to further embodiments, the grouping step requires that all task data objects belonging to the same task data object group share the same value or value range of two or more attributes. Each task data object group comprises one or more task data objects. The term 'ID' and 'identifier' are herein used synonymously.

For at least some of the task data object groups, all task data objects contained in a task data object group are aggregated to provide aggregated task data object groups. Each aggregated task data object group comprises an aggregated data value, said aggregated data value being calculated from all task data objects being contained in said aggregated task data object group. The calculation uses all or parts of the data contained in each task data object belonging to said aggregated task data object group. For each task data object group to be aggregated, one aggregated task data object group comprising the task data objects of said task data object group is provided.

For example, in case the attribute used in the grouping step was an ID of a lab-device, the aggregated data value can be the number of tasks to be executed by the lab-device having assigned the device-ID. In case two attributes were used in the grouping step, e.g. a user-ID and a device-ID, each task data object group comprises only task data objects being indicative of a task to be executed by one particular user identified by said user-ID and by one particular lab-device identified by said device-ID. In this case, the aggregated data value can be, for example, the number of tasks to be executed by the user having assigned the user-ID on the lab-device having assigned the device-ID. The aggregated data value may also be, for example, a position information, e.g. a room-ID being indicative of a storage room comprising the reagents and/or consumables specified in a set of aggregated task data objects.

A graphical user interface element (GUI element) is specified for each of the aggregated task data object groups whereby the aggregated data value calculated for each of said aggregated task data object group is used for specifying the GUI element of said aggregated group, wherein each of the specified GUI elements is representative of said aggregated task data object group. Each of the specified GUI I elements is displayed on a graphical user interface, thereby providing an aggregated view.

An '**aggregated view**' is a view provided to a user, e.g. via a graphical user interface, on data having been aggregated for a particular group of data objects. An aggregated view presents some or all data contained in the aggregated data objects in a condensed summary form, thereby providing the user with an intuitive and quickly comprehensible presentation of all or some of the data contained in a multitude of aggregated data objects. Providing an aggregated view can comprise, for example, displaying the number of data object aggregated, displaying an aggregated priority score, or displaying any other form of aggregated data value. The aggregated data value may be displayed as alphanumerical character, may be encoded by a color schema or may be encoded by using a set of predefined images.

A GUI element is a data object some of which's attributes specify the shape, layout and/or behavior of an area displayed on a graphical user interface, e.g. a screen. A GUI element can be a standard GUI element such as a button, a text box, a tab, an icon, a text field, a pane, a check-box item or item group or the like. A GUI element can likewise be an image, a displayed alphanumeric character or any combination thereof. Using an aggregated data value for specifying a GUI element implies that at least some of the properties of the displayed GUI elements depend on said aggregated data value.

According to embodiments of the invention wherein a user-ID is used for grouping task data objects, the GUI element representing an aggregated task data object group can comprise a numerical value being indicative of the total number of tasks to be executed by the user being identified by said user-ID.

According to embodiments of the invention wherein a device-ID is used for grouping task data objects, the GUI element representing an aggregated task data object group can comprise a numerical value being indicative of the total number of tasks to be executed by the lab-device being identified by said device-ID. Alternatively, or in addition, the GUI element representing an aggregated task data object group can be an image being indicative of the lab-device or type of the lab-device whose device-id is assigned to the aggregated task data objects, thereby facilitating a user the identification of the lab-device where the tasks indicated by the aggregated task data objects are to be performed.

According to embodiments using a device-component-ID for grouping task data objects, the GUI element representing an aggregated task data object group can comprise a number being indicative of the total number of tasks to be executed by or using a lab-device-component being identified by said device-component-ID.

Alternatively, or in addition, the GUI element representing an aggregated task data object group can be indicative of the lab-device-component having assigned said device-component-ID. By highlighting or otherwise optically accentuating said identified device-component where a task needs to be performed, the identification of said lab-device-component by a user for performing the tasks indicated by the task data objects is greatly facilitated.

According to embodiments of the invention, an analyzer comprises one analysis component and one or more reagent components, each reagent component comprising at least one reagent container. In case the reagent container of the first reagent component runs out of reagent, the grouping and aggregation of task data objects according to the attribute 'device-component-ID' results in the provision of an aggregated view: the analysis system is represented and displayed on a graphical user interface as a schematic diagram, the schematic diagram representing the analyzer- and reagent components as distinct areas. According to said embodiments, the task of refilling the reagent of the first reagent component is represented as a task data object. Said task data object has assigned the device-component-ID of the first reagent component. Upon grouping and aggregating task data objects according to their assigned device-component-IDs, the aggregated task data object groups being indicative of tasks to be performed on or by the first reagent component are represented as GUI element by highlighting the area of the schematic analyzer diagram representing the first reagent component. As a result, the highlighted area of the schematic analyzer diagram provides a user with an aggregated view on tasks which need to be performed on or by the highlighted first reagent component of said analyzer.

The aggregated data value of each aggregated task data object group is used for specifying the GUI element representing said aggregated task data object group. Using the aggregated data value for specifying a GUI element can, but does not necessarily have to, imply displaying an aggregated numerical data value, e.g. the total number of tasks indicated by all task data objects belonging to the aggregated task data object group. Likewise, the value of the aggregated data value can be used to specify a GUI element having a particular design e.g. by using different colors or images. For example, providing an aggregated view of a large number of tasks can comprise displaying GUI elements of a different color compared to providing an aggregated view of a small number of tasks. Likewise, providing an aggregated view of a set of highly urgent tasks can involve the specification of GUI elements having a different color than the color used for the specification of GUI elements representing a set of routine tasks with low priority.

A value range can comprise a range of user-IDs, a range of device-IDs and the like. Likewise, the position of a lab-device can be encoded as a two component position-ID. Laboratory A may comprise three laboratory rooms identified e.g. as position-ID 'A.1', 'A.2' and 'A.3' while Laboratory B may comprise two laboratory rooms identified e.g. as 'B.1' and 'B.2'. Depending on the embodiment of the invention, task data objects having assigned a position-ID may be grouped according to the first component of the position-ID (A or B) or according to the full position-ID (e.g. A.2, B.1). By using either the first component of the position-ID or the full position-ID as attribute in the grouping step, a coarse-grained or a fine-grained grouping and aggregation of task data objects based on an assigned position-ID can be accomplished.

Providing a user with an aggregated view of tasks to be executed in a laboratory is highly advantageous as it allows organizing and performing tasks in the context of a laboratory more efficiently and helps avoiding errors. A user may, for example, have assigned several hundred task data objects, many of them being indicative of completely different tasks. For example, a user may have assigned a first set of task data objects being indicative of the task of manually evaluating the plausibility of measurement values obtained by an analyzer on a set of samples. The user may have assigned a second set of task data objects being indicative of the task to replace empty reagent bottles in various lab-devices. Said user may have in addition assigned a third set of task data objects being indicative of highly urgent tasks such as restarting an erroneous lab-device blocking a pipeline of samples to be processed. Said user may in addition have assigned fourth and fifth sets of task data objects being indicative of other, not as urgent routine tasks. A plain list of tasks would confuse a user due to the sheer number and diversity of tasks to be executed.

An aggregated view helps to reduce complexity by displaying a user only a limited amount of information at the same time, thereby reducing the risk that a user overlooks a critical task.

Aggregating data contained in a multitude of task data objects assists a user in performing his tasks more efficiently. For example, by grouping task data objects being indicative of fetching reagent bottles from a particular storage room into one task data object group, redundant walks and inefficient workflow steps can be eliminated: a user is provided with an aggregated view of a multitude of reagents and consumables to be fetched from a particular room, thereby allowing the user to avoid redundant walks to said storage room. Likewise, if multiple tasks related to repair works have to be performed on various lab-devices, an aggregated view of tasks data objects guarantees that a user visiting a particular lab-device is aware of tasks related to said particular lab-device.

In a further advantageous aspect, embodiments of the invention used as a laboratory workflow management system reduce the time to learn because the user does not have to look for and remember which screen to access in order to perform a particular task. Rather, necessary tasks and/or tasks having been assigned to a user are dynamically determined by the analysis system and presented to the user in the form of an easily comprehensible, aggregated view.

According to further embodiments of the invention, the method further comprises the step of representing each received task data object as a node of a hierarchical drill-down graph, the hierarchical drill-down graph comprising at least two hierarchical levels. According to said embodiments, the grouping step comprises determining one grouping node of the hierarchical drill-down graph, the grouping node being a starting point for executing a drill-down analysis. The drill-down analysis is a data aggregation operation executed on the grouping node and all direct and indirect successor nodes of the grouping node. All task data objects being grouped into the same task data object group share the same attribute value or attribute value range of at least one attribute.

According to further embodiments of the invention, the steps of grouping and aggregating task data objects can be performed on multiple hierarchical levels of a drill-down graph by selecting a particular node in said drill-down graph. Said embodiments are advantageous, as they provide a user with multiple levels of data aggregation and with means to specify for which node, including its direct and indirect successor nodes, a drill-down analysis shall be executed.

A drill-down graph is a data structure comprising data objects being represented as nodes and edges of a graph. A drill-down graph allows the execution of a drill-down analysis of the data contained in its nodes, wherein the pathways that can be used in the drill-down analysis are specified by the drill-down graph's edges.

According to embodiments of the invention, the drill-down graph comprises a first set of nodes which do not represent task data objects. Each of the received task data object is represented as node and added to the drill-down graph by creating new edges, said new edges connecting at least some of the first nodes with the newly created nodes representing the received task data objects.

According to some embodiments of the invention, all task data objects are represented as leaf nodes of the drill-down graph. According to further embodiments of the invention, at least some of the received task data objects are represented as non-leave nodes.

Depending on the embodiment of the invention, the hierarchical drill-down graph comprises two or more hierarchical levels. Each node of the drill-down graph not being a leaf node comprises one or more child nodes. At least some nodes in the drill-down graph can be used as starting point for executing a drill-down analysis. The drill-down analysis is a data aggregation operation executed upon selection of a node as starting node for the drill-down analysis. Said node is referred to as 'grouping node'. The data contained in the grouping node and in its direct and indirect successor nodes is considered and aggregated during said drill-down analysis. A 'direct successor node' of a node X is a child node of said node X. An 'indirect successor node' is any node S that can be reached starting from said node X by traversing the graph downwards, wherein S is not a child node of node X.

According to embodiments of the invention, a drill-down analysis executed for a particular grouping node comprises the steps of:
- grouping all task data objects being represented by the grouping node or by a direct or indirect successor node of the grouping node into task data object groups, wherein all task data objects belonging to the same task data object group share the same attribute value or attribute value range of the at least one attribute,
- for at least some of the task data object groups, aggregating all task data objects contained in a task data object group to provide aggregated task data object groups, each aggregated task data object group comprising an aggregated data value, said aggregated data value being calculated from all task data objects being contained in said aggregated task data object group,
- specifying a GUI element for each of the aggregated task data object groups, whereby the aggregated data value calculated for said aggregated task data object group is used for specifying said GUI element, said specified GUI element being representative of said task data object group, and
- displaying the specified GUI elements on a graphical user interface for providing an aggregated view.

According to further embodiments of the invention, the method further comprises the steps of:
- displaying at least one navigation GUI element, wherein the at least one navigation GUI element is a selectable pointer to a node of the drill-down graph,
- upon selection of said at least one navigation GUI element by a user, using the node being pointed at by the selected navigation GUI element as grouping node. The at least one navigation GUI element provides a user with the option to specify the starting point for executing a drill-down analysis.
- triggering the execution of a drill-down analysis for the used grouping node. As a result, the user is provided an aggregated view on data contained in task data objects which are represented as direct or indirect successor nodes of the grouping node used in the drill-down analysis.

According to embodiments of the invention, a 'navigation GUI element' can be any kind of selectable GUI element, e.g. a button, an icon or the like. According to preferred embodiments, the navigation GUI element has an asymmetric shape capable of indicating a direction. Such an asymmetric shape can be, for example, an arrow, a triangle, a U-form shaped element, or the like. According to further embodiments, at least some of the non-leaf nodes of the drill-down graph have assigned a name and each navigation GUI elements displays the name of the node it points at.

According to further embodiments of the invention, the method further comprises the step of displaying two or more navigation GUI elements on the GUI. The displayed navigation GUI elements constitute a navigation path, the navigation GUI elements of said navigation path connecting nodes having already been used as grouping node in a drill-down analysis. The navigation path allows a user to navigate back and forward between nodes of the drill-down graph by selecting navigation GUI elements pointing to said nodes. The last navigation GUI element in said navigation path is a pointer to a node having been used as grouping node for executing a drill-down analysis whose resulting aggregated view is currently displayed.

According to preferred embodiments of the invention, the navigation GUI elements have an asymmetric shape and are arranged on the GUI in a way that the direction indicated by said navigation GUI elements is the same, e.g. from left to right, and that as a result the direction of the navigation path from the root node to the node used as grouping node in the current drill-down analysis is displayed.

According to embodiments of the invention, the first navigation GUI element of said navigation path points to the root node of the drill-down graph.

According to embodiments of the invention, the last navigation GUI element of said navigation path is not selectable, thereby preventing the user from triggering the current drill-down analysis a second time.

According to further embodiments, the method further comprises the steps of:
- storing, for each drill-down analysis, at least the aggregated data value of each aggregated task data object group in a drill-down history,
- selecting a navigation GUI element by a user, the user thereby selecting a grouping node, the grouping node corresponding to a previously executed drill-down analysis, and
- loading the stored aggregated data values aggregated in said previously executed drill-down analysis, thereby making a re-execution of the drill-down analysis for the selected grouping node unnecessary.

A drill-down history is a data structure being stored in the working memory of a computer executing the drill-down analysis. By using said drill-down history, moving forward and backward in the navigation path is significantly accelerated: usually, memory access times of working memories are very short. In addition, the aggregated results can be read from the drill-down history without repeating the potentially time consuming aggregation step. In order to guarantee that for each node having been used at least once as grouping node the generated one or more aggregated data values can be loaded, said one or more aggregated data values have to be stored in association with the node used as grouping node in the corresponding drill-down analysis.

According to further embodiments of the invention, the method further comprises the steps of:
- specifying one or more categories,
- representing each of the one or more categories as a selectable category GUI element, wherein said selectable category GUI element can be in particular a tab, but can also be a button, an icon or the like,

- creating, for each category, a corresponding drill-down graph,
- adding each of the one or more nodes representing a received task data object to each of the created one or more drill-down graphs,
- upon selection of one of the one or more category GUI elements by a user, selecting the drill-down graph corresponding to the selected category GUI element, and
- executing a drill-down analysis, wherein the step of grouping task data objects and aggregating data is executed according to the graph topology of the selected drill-down graph.

The term '**category**' as used herein refers to an information architecture providing a particular view on the data contained in the received task data objects. Each category corresponds to a drill-down graph. Depending on the embodiment of the invention, the topology of each drill-down graph corresponding to a particular category may be unique, thereby providing a unique graph topology for grouping, aggregating and interpreting data contained in the received task data objects.

Using multiple different categories corresponding to multiple different drill-down graphs is particularly advantageous as a user is thereby provided with different entry points to work on the received task data objects and is provided with the option to organize and aggregate tasks according to different schemas.

According to embodiments of the invention, each category is selected from a group comprising a 'Routine' category, a 'Lab' category and a 'Utility' category.

The 'Routine' category encompasses data being related to one or more biological samples and data being related to the production of analytical results and/or data providing a user with the option to monitor and/or control the execution of a laboratory procedure on one or more biological sample.

The 'Lab' category can be used to organize data according to one or more lab-devices existing in a laboratory. Views provided according to the 'Lab' category provide a user with the option to monitor and/or control the status of one or more lab-devices, consumables, reagents and/or waste. For example, the filling level of a reagent, error states of a lab-device, or the like can be monitored and the affected device can be started or stopped by the user accordingly.

The 'Utility' category comprises configuration data which can be used for configuring lab-devices, user-roles or user-permissions. The configuration data can also comprise configuration data for of one or more tests to be performed on one or more samples, user management related configuration data and/or log entries.

According to further embodiments of the invention, additional and/or other categories are used to structure and aggregate data contained in the received task data objects.

According to further embodiments of the invention, the method further comprises creating at least one link, the at least one link connecting a first node with a second node, wherein the first node belongs to a first drill-down graph, the first drill-down graph corresponding to a first category, and wherein the second node belongs to the first drill-down graph or to a second drill-down graph, the second drill-down graph corresponding to a second category. In case the second node belongs to the second drill-down graph, the link connects the first and the second drill-down graph and provides a user with the option to navigate between drill-down graphs of different categories.

According to preferred embodiments of the invention, the links are specified by a business analyst, not by an end-user. The usage of links to connect nodes is particular advantageous for the following reasons:
- unlike edges, a link can connect nodes belonging to different drill-down graphs, thereby allowing the user to navigate between different drill-down graphs.
- if a user wants to change context, e.g. wants to view data of one or more groups of task data objects created by grouping and aggregating said task data objects according to different drill-down graphs, a link allows a user to switch between different aggregated views, each aggregated view being generated based on the topology of a different drill-down graph.
- a link allows connecting nodes belonging to distant hierarchical levels being separated from each other by one or more other hierarchical levels, thereby allowing a user to skip executing drill-down analyses for each intermediate hierarchical level.
- a link, which is not an edge of one of the drill-down graphs, allows the introduction of circular navigation paths. As a result, a user can be provided with almost unlimited freedom of navigation within or between multiple drill-down graphs while at the same time the generation of cyclic pathways in the drill-down graph which may lead to inconsistencies is prohibited: the topology of a drill-down graph is constituted solely by its nodes and edges, not by the links.

According to further embodiments of the invention, one or more user-IDs and/or role-IDs are assigned to each of the received task data objects. A user-ID and/or role-ID of a user are received upon a login of said user. Upon receipt of said user-ID and/or role-ID, only task data objects are used for executing a drill-down analysis which have assigned the received user-ID and/or role-ID.

Depending on the embodiment of the invention, a user may log into the analysis system by a multitude of ways, e.g. by keying in a passphrase and/or providing biometric data such as a fingerprint. In accordance with an embodiment of the invention the user identification is performed by means of an RFID method and/or by means of a chip card. In particular, a mobile device can be used for the user identification as the mobile device typically signals the user's telephone number when a communication channel is established between mobile device and server computer which can be used for the user's identification. Each user has assigned a user-ID which is an identifier being unique for said user. A user may have assigned a role-ID in addition or instead of a user-ID. A role ID is a unique identifier for a group of one or more users fulfilling a particular role in a laboratory and having assigned role specific rights and duties.

Depending on the embodiment of the invention, the user can log into the analysis system via a man-machine interface provided by the server computer, being provided by a lab-device connected to the server computer and/or being provided by any other computer or a mobile device connected to the server computer via a network.

Upon a login-event received from the server computer, an aggregated view is provided to the user, the aggregated view having been created by selectively using only task data objects in the grouping and data aggregation step which have assigned the user-ID and/or role-ID of the user received upon the log-in event.

According to further embodiments, one or more user-IDs and/or role-IDs are assigned to each of the received task data objects and the method for providing an aggregated view further comprises the steps of:
- receiving a user-ID and/or role-ID of a user upon a login of said user, and
- selectively using only task data objects for executing the grouping step and the aggregation step which have assigned the received user-ID and/or role-ID.

According to further embodiments of the invention, each task data object is assigned an urgency level attribute. Some of said embodiments further comprise the step of determining that no user is currently logged-in, and grouping and aggregating task data objects according to the value of the assigned urgency level attribute. In the aggregation step, each aggregated task data object group is assigned an aggregated urgency level value. Said aggregated urgency level value is calculated as the maximum urgency level attribute value of any of the task data objects within said aggregated task data object group. Only GUI elements of those groups of task data objects are displayed whose aggregated urgency level value is above a threshold. As a result, even in case no user is logged-in, an aggregated view on the most urgent tasks to be executed in order to preserve the operability of the analysis system is provided.

According to further embodiments of the invention, the method further comprises the steps of
- specifying a favorite GUI element, the favorite GUI element being a user-specific, selectable pointer to a node within the drill-down graph,
- displaying the specified GUI element, wherein upon selection of said favorite GUI element, the execution of a drill-down analysis is executed, wherein in said execution the node being pointed at by the favorite GUI element is used as grouping node.

A 'favorite GUI element' can be, for example, a selectable button, icon or any other selectable GUI element which can be displayed on a graphical user interface. Favorite GUI elements allow users to navigate directly to a certain node in a drill-down graph in order to obtain an aggregated view of task data objects according to a drill-down analysis being based on using said node as grouping node.

According to further embodiments of the invention, the method further comprises the steps of:
- providing each of the GUI elements according to a first GUI element version, a GUI element version corresponding to a particular graphical design, the first GUI element version being adapted to be displayed on a small screen, in particular a screen of a mobile user device, wherein each GUI element is a pointer to a node in the drill-down graph,
- displaying first GUI elements in a first pane, the first pane approximately being of screen size,
- upon selection of one of said first GUI elements, using the node being pointed at by the selected GUI element as grouping node and executing a drill-down analysis by executing steps B-E,
wherein the GUI elements displayed in step E are second GUI elements, the second GUI elements being displayed in a second pane, the second pane approximately being of screen size, the second pane replacing the first pane.

A GUI element version corresponding to a particular graphical design is a specification of the size, shape and other features determining the appearance and dynamic behavior of a GUI element on a graphical user interface.

Said embodiments are particularly advantageous as they provide an aggregated view of data which is particularly adapted to be displayed on a small screen, e.g. of a mobile device of a user such as a smart phone. Some of said embodiments can be used as emergency systems allowing the notification of a user with appropriate permissions and skills, e.g. a lab technician, whenever a highly urgent tasks needs to be executed immediately in order to preserve the operability of an analysis system.

According to embodiments of the invention, a technician can be notified on the status of a lab-device by the server computer via the push method. The server computer continuously monitors status information of the lab-devices belonging to an analysis system. In dependence on the status information, one or more task data objects are created and received by the server computer. The step of grouping and aggregating task data objects is performed by using at least an urgency level attribute assigned to each of the received task data objects as attribute for grouping. In case the aggregated urgency level value assigned to any of the groups of task data objects exceeds a threshold value, one or more users are notified of the urgent tasks. The notification is executed by the server computer sending a specification of an aggregated view on said urgent tasks to the mobile device of a user. Said user can be a default user, e.g. the responsible lab technician. Said user can according to further embodiments likewise be a user who is identified by a user-ID, said user-ID being assigned to each of the aggregated task data objects.

As the size of a mobile device is usually small, it is advantageous to display the results of a drill-down analysis according to a first display mode, the first display mode using panes being approximately of screen size, the first display mode using GUI elements corresponding to a first version of GUI elements. Said display mode can also be considered as 'full screen mode'. GUI elements corresponding to the first version of GUI elements are small enough to be displayed on a small screen such as a screen of a mobile device.

A drill-down analysis is executed upon selecting a GUI element of the first pane. The aggregated data obtained as a result of said drill-down analysis is displayed on a second pane. The first pane and the second pane cannot be viewed by the user at the same time. Rather, the second pane replaces the first pane.

As the size of screens used in current computer systems and/or current analysis systems is usually large enough to allow the simultaneous display of multiple GUI elements at the same time without confusing the user, it is advantageous to display the results of a drill-down analysis on said graphical user interfaces according to a second display mode. The second display mode uses panes being approximately of half screen size. Said display mode can also be considered as 'detaileddetailed display mode'. GUI elements belonging to a second version of GUI elements are of appropriate size to be displayed on a medium sized screen such as a screen of a current computer system. A drill-down analysis is executed upon selecting a GUI element of the first pane. The aggregated data obtained as a result of said drill-down analysis is displayed on a second pane. The first pane and the second pane can be viewed by the user according to the second display mode at the same time.

According to further embodiments of the invention, the method further comprises the steps of:
- providing each of the GUI elements according to a second GUI element version, a GUI element version corresponding to a particular graphical design, the second GUI element version being adapted to be displayed on a medium-sized screen, in particular a screen of a computer system or a screen of a lab-device, wherein each GUI element is a pointer to a node in the drill-down graph,
- displaying first GUI elements in a first pane, the first pane approximately being of half screen size,
- upon selection of one of said first GUI elements, using the node being pointed at by the selected GUI element as grouping node and executing a drill-down analysis by executing steps B-E,
- wherein the GUI elements displayed in step E are second GUI elements, the second GUI elements being displayed in a second pane, the second pane approximately being of half screen size, the first pane and the second pane being displayed at the same time.

According to further embodiments of the invention, previously described embodiments of said method for providing an aggregated view are implemented by an analysis system for analyzing biological samples, the analysis system comprising one or more lab-devices. According to further embodiments of the invention, the method comprises the steps of identifying a user by one of said one or more lab-devices, sending of a user identifier of the identified user and a device identifier of the one of the first and second lab-devices to a server computer, determining a task to be executed by the identified user by the server computer, the server computer thereby using the user identifier and the device identifier, and sending a signal comprising task data objects being indicative of the determined task from the server computer to the lab-device.

According to embodiments of the invention, a grouping threshold can be specified, e.g. by a business analyst. A grouping threshold is the minimum number of task data objects that need to be contained within a task data object group in order to determine an aggregated data value and to use said data value for specifying a GUI element representing an aggregated task data object group. According to said embodiments, the number of task data objects in a current task data object group is determined. In case the determined number exceeds the grouping threshold, a drill-down analysis is executed and a user is provided with an aggregated view. In case it is determined that the current number of task data objects does not exceed the grouping threshold, each single task data object is graphically represented in the form of a separate GUI element.

According to still further embodiments of the invention, one or more of the GUI elements representing an aggregated task data object group are link elements displayed in a separate area of the screen which is herein referred to as 'tasks area'. Each link element provides a user with access to program instructions for performing the one or more tasks represented by said link element. For example, if 12 task data objects each indicate the task of manually evaluating the plausibility of a measurement value obtained as analysis result, a link element representing said 12 task data objects comprises a link which, when selected, triggers the display of a more detailed view, said view containing data required for executing said evaluation.

For example, upon selection of said link element, calibration data of the analytical device used for obtaining the measurement values is displayed, said calibration data being required for evaluating the plausibility of an obtained measurement value.

According to further embodiments, selecting a link element by a user triggers the execution of a laboratory procedure on one or more samples by a lab-device and/or triggers the display of one or more selectable GUI elements for controlling, e.g. starting and/or stopping said lab-device.

According to embodiments of the invention, each task data object comprises at least a first and a second attribute. In case a first grouping node is selected, the first attribute is used for executing a first drill-down analysis comprising the execution of steps B-E. In case a second grouping node is selected, the second attribute is used for executing a second drill-down analysis comprising the execution of steps B-E, the first and the second attribute not being identical.

For example, during the execution of a first grill-down analysis, a user-ID may be used as attribute for grouping and aggregating task data objects. In a second drill-down analysis executed e.g. for a particular group of task data objects all having been assigned to a particular user-ID, the device-ID can be used as attribute for grouping and aggregating all task data objects having been assigned said user-ID. Depending on the embodiment of the invention, the attribute or attributes used in the grouping step may be predefined, specified by a business-analyst and/or specified by an end-user. As a result, the user is provided with an analytical system and method providing an aggregated view on data of task data objects grouped and aggregated according to attributes which can flexibly be adjusted to the particular requirements of a laboratory.

According to embodiments of the invention, the status of the at least one lab-device is monitored by the server computer. The server computer dynamically creates or receives new task data objects and/or modifies or deletes at least some of the received task data objects in dependence on the received status information. Upon creating, modifying or deleting a received task data object the re-execution of a drill-down analysis is triggered. As a result of said re-execution, a dynamically updated, aggregated view is provided to the user.

In accordance with further embodiments of the invention an analysis system for analyzing biological samples is provided, the analysis system comprising at least one lab-device and a server computer, the server computer having a server interface component for receiving task data objects. Each task data object comprises at least one attribute. At least some of the received task data objects are indicative of a laboratory procedure to be performed on at least one biological sample using the at least one lab-device. The server computer comprises a processing component for grouping the task data objects into task data object groups wherein all task data objects belonging to the same task data object group share the same attribute value or attribute value range of the at least one attribute. For at least some of the task data object groups, all task data objects contained in a task data object group are aggregated to provide aggregated task data object groups, each aggregated task data object group comprising an aggregated data value, said aggregated data value being calculated from all task data objects being contained in said aggregated task data object group. For each of the aggregated task data object groups a GUI element is specified, whereby the aggregated data value calculated for said aggregated task data object group is used for specifying said GUI element, said specified GUI element being representative of said task data object group. The analysis system further comprises at least one graphical user interface for displaying the specified GUI elements for providing an aggregated view.

Depending on the embodiment, the graphical user interface can be part of a lab-device, can be part of the server computer system, and/or can be part of any other processing device being connected to the server computer via a network. Said other processing device can be, for example, a mobile device of a user or a computer connected to the intranet of the laboratory.

According to further embodiments of the invention, a device-ID is assigned to at least some of the received task data objects, the device-ID being indicative of one of the at least one lab-devices. At least one lab-device belonging to the analysis system comprises a device identification component for identifying said lab-device at the server computer via the lab-device's device-ID. According to said embodiments, the server computer comprises a service interface component. The server interface component is operable to send a signal to the identified lab-device, the signal comprising data having been aggregated on all received task data objects to which was assigned the device-ID of the identified lab-device. As a result, a user working on said lab-device can constantly be kept up to date regarding the tasks that need to be executed in the laboratory, in particular urgent tasks.

According to further embodiments of the invention, said device-ID is indicative of a mobile device of the user, the server computer is operable to identify the mobile device via its device-ID, e.g. the mobile number of the user. The server interface component is operable to send a signal to the identified mobile device, the signal comprising data having been aggregated on all received task data objects to which was assigned the user-ID of the user of the identified mobile device. As a result, a user can constantly be kept up to date regarding the tasks that need to be executed in the laboratory, in particular urgent tasks, via his mobile device.

According to further embodiments of the invention, a user-ID and/or role-ID is assigned to at least some of the received task data objects, each user-ID being indicative of one user, each role-ID being indicative of a role assigned to at least one user. The at least one lab-device comprises a user identification component for identifying a user by the user's user-ID and/or role-ID and an interface component for sending the user-ID and/or role-ID of the identified user. The server computer comprises a service interface component, wherein the server interface component is operable to send a signal to the lab-device identified by the device-ID, the signal comprising data aggregated on received task data objects to which is assigned the user-ID and/or role-ID of a user identified by the lab-device. According to further embodiments, the server computer is in addition operable to send said signal to a mobile device of the identified user.

According to still further embodiments, the user can identify himself at the server computer via a graphical user interface provided by the server computer. Said graphical user interface can be a screen of the server computer or can be a monitor screen of a further computer system, the further computer system being connected to the server computer via a network, e.g. the intranet of a laboratory.

In accordance with further embodiments of the invention an analysis system for analyzing biological samples is provided comprising at least one lab-device, the at least one of the lab-device having a user identification component for identifying a user, a device identification component for identifying the lab-device, and an interface component for sending a user identifier of the identified user and a device identifier of the lab-device, and a server computer having a server interface component for receiving the user identifier and the device identifier, a processing component for determining a task to be executed by the identified user, wherein the server interface component is operable to send a signal to the lab-device identified by the device-ID via the service interface component, the signal comprising data aggregated on received task data objects which have assigned the user-ID and/or role-ID of the user identified by the lab-device.

Embodiments of the invention are particularly advantageous as the user is informed regarding one or more tasks that need to be executed by that user by the server computer upon logging in using the at least one lab-device. This enables a highly convenient and efficient operation of the analysis system.

In accordance with an embodiment of the invention completion of the execution of a task is signalled to the server computer. For example, the user can use a lab-device for execution of the determined task and then enter an acknowledgement into said lab-device such that completion of the execution is signalled from the lab-device to the server computer.

In accordance with an embodiment of the invention the server computer comprises a database for assigning a first set of tasks to the first lab-device and for assigning a second set of tasks to the second lab-device. The processing component of the server computer generates a message when the determined task is not assigned to the identified lab-device in the database. For example, the message is sent from the server computer to the identified lab-device indicating another one of the lab-devices to which the determined task is assigned and which can thus be used by the user for execution of the determined task.

In accordance with an embodiment of the invention the analysis system further comprises at least one mobile device. The mobile device can receive the signal being indicative of the determined task. This signal is also indicative of one of the lab-devices to be used for execution of the determined task. This is particularly convenient and efficient as the integration of at least one mobile device into the analysis system allows ubiquitous operation of the analysis system.

According to further embodiments of the invention, the method comprises the steps of identifying a user by a lab-device, sending a user identifier of the identified user and a device identifier of the lab-device to a server computer, determining at least one task data object by the server computer using the user identifier and the device identifier, the at least one task data object being indicative of the determined task , and sending a signal comprising the task data objects from the server computer to the one of the lab-devices.

The determination of the task by the server computer can be performed using a workflow management system that runs on the server computer or on another server computer that is connected to the network.

In accordance with an embodiment of the invention the mobile device of the analysis system receives a signal comprising task data objects being indicative of the at least one determined task and also being indicative of the lab-device to be used for execution of this task. In addition the task data objects are indicative of the position of the lab-device to be used for execution. The position information can comprise an indication of the geographic and/or topological location of the lab-device. For example, the position information is provided as a laboratory room number for indicating the laboratory room in which the lab-device is located.

In accordance with an embodiment of the invention the task data objects contained in the signal received from the server are indicative of a priority of the tasks. In response to receipt of the signal by the mobile device or the lab-device the tasks are grouped and aggregated and an aggregated urgency level is calculated for each aggregated task data object group, the aggregated task data objects being represented by a displayed GUI element only in case the aggregated urgency level of an aggregated task data object group exceeds a threshold value.

According to further embodiments of the invention, the analysis system further comprises at least one mobile device. The mobile device receives the signal being indicative of the at least one determined task, the signal being indicative of one of the lab-devices to be used for execution of the at least one task.
For example, the mobile device can be in compliance with a mobile digital telecommunication standard, such as a GSM standard, such that the mobile device has an integrated card reader for a so-called subscriber identity module (SIM) card. The data that is stored on the SIM card can be used for the user identification.

According to further embodiments of the invention, the task data objects contained in the signal are indicative of position information indicating a geographic or topological location of the lab-device to be used for the execution of the determined task.

According to further embodiments of the invention, the mobile device upon completion of at least one determined task sends a message indicating completion of said task to the server computer.

In still another aspect the present invention relates to a computer program product comprising executable program instructions for execution of a method by a server computer of an analysis system, said method being a method for providing an aggregated view according to any of the embodiments of said method described previously.

In accordance with embodiments of the invention, a computer-readable, non-transitory storage medium having stored therein instructions that when executed by a processor cause the processor to group and aggregate task data objects according to any of the previously described embodiments of the invention is provided.

### Brief description of the drawings

In the following, embodiments of the invention are explained in greater detail by way of example, only making reference to the drawings in which:
- Figure 1: is a flow chart of a method providing an aggregated view,
- Figure 2: depicts a screenshot of a graphical user interface (GUI) when no user is logged in,
- Figure 3: depicts a screenshot of a login dialog provided to a user,
- Figure 4a: is a schematic drawing of an overview pane,
- Figure 4b: depicts the overview pane displaying user specific data,
- Figures 5a: depicts an overview on available lab-devices displayed upon selection of the 'Lab' category tab,
- Figure 5b: depicts an aggregated view on consumables, reagents and other items used by the lab-device 'Cavallo', the aggregated view being the result of a drill-down analysis having been executed for said lab-device,
- Figures 6a-6c: illustrate the dynamic specification and aggregated display of tasks,
- Figure 7a: depicts an aggregated view generated as the result of a drill-down analysis to the user according to a first display mode,
- Figure 7b: depicts an aggregated view generated as the result of a drill-down analysis to the user according to a second display mode,
- Figure 8a: depicts the screen of a mobile device displaying a first pane according to a first display mode, the first pane comprising a tasks pane and a favorites pane,
- Figure 8b: depicts the screen of the mobile device displaying a second pane according to the first display mode, the second pane providing an aggregated view generated as the result of a drill-down analysis,
- Figure 9: depicts parts of two different drill-down graphs corresponding to the categories 'Routine' and 'Lab', the drill-down graphs being connected via a link,
- Figure 10a: is a detailed illustration of the drill-down graph corresponding to the category 'Routine',
- Figure 10b: illustrates the relation between navigational path displayed to the user and the topology of the corresponding drill-down graph,
- Figure 11: is a block diagram of an embodiment of an analysis system of the invention, and
- Figure 12: is a block diagram of an embodiment of an analysis system of the invention where the task of refilling a reagent is signalled to a user.

### Detailed description

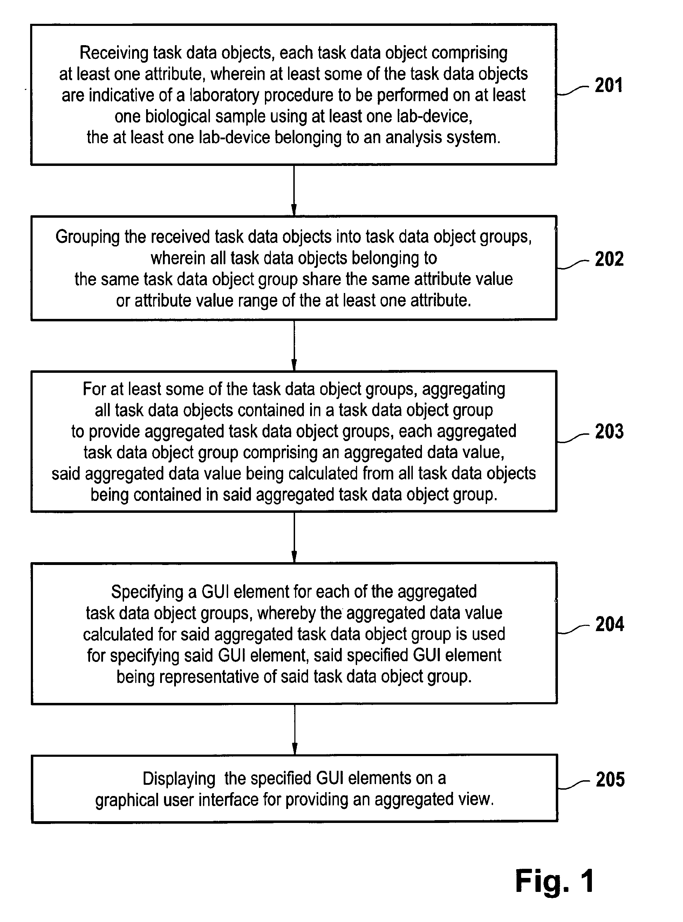

Figure 1 is a flowchart of a method providing an aggregated view to a user. In step 201 task data objects are received, e.g. by a server computer. At least some of the received task data objects are indicative of a laboratory procedure to be performed on at least one biological sample using at least one lab-device.
The received task data objects are grouped into task data object groups. All task data objects belonging to the same task data object group share the same attribute value or attribute value range of the at least one attribute.

In step 203 for at least some of the task data object groups, all task data objects of said task data object group are aggregated to provide aggregated task data object groups, each aggregated task data object group comprising an aggregated data value, said aggregated data value being calculated from all task data objects being contained in said aggregated task data object group.

In step 204 for each of the aggregated task data object groups a GUI element is specified, whereby the aggregated data value calculated for said aggregated task data object group is used for specifying said GUI element, said specified GUI element being representative of said task data object group.

In step 205 the specified GUI elements are displayed on a graphical user interface, thereby providing a user with an aggregated view of data contained in the aggregated task data objects.

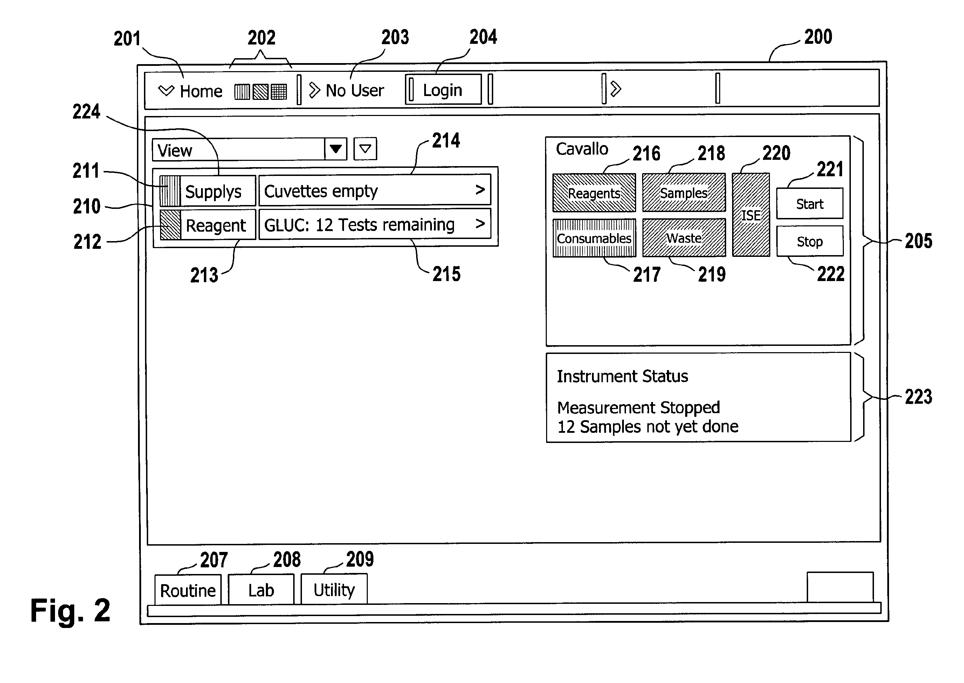

Figure 2 depicts a screenshot of a graphical user interface (GUI) when no user is logged in. The GUI element 203 indicates that currently no user is logged in. The selectable GUI element 204 triggers, upon selection, the display of a log-in dialog window 300 to the user.

The overview pane 201 comprises a tasks area 210, a lab-device area 205 and a lab-device status area 223. Lab-device area 205 displays information on the lab-device "Cavallo" in greater detail. Cavallo comprises multiple logical, functional and/or physical subunits: a reagents subunit represented by GUI element 216 comprises one or more reagents for executing a particular analysis; consumables subunit represented by GUI element 217 comprises consumables such as disposable cuvettes or pipette tips; waste subunit is represented by GUI element 219 and a samples subunit for receiving and/or storing biological samples is represented by GUI element 218. Selectable GUI elements 221, 222 are displayed and allow a user to start or stop the lab-device Cavallo.

The GUI elements 221, 222 allow a user to control the operation of the lab-device via the GUI in dependence on the dynamically displayed status information on the lab-device and/or tasks to be performed by said lab-device. The lab-device status area 223 provides a user with an aggregated view on tasks to be performed by one or more lab-devices or by one or more subunits of a lab-device. If one or more tasks needs to be performed by or in respect to one of said subunits, said subunit is highlighted. In the depicted embodiment, the GUI element 217 representing the "consumables" subunit comprising the empty cuvette container is assigned a red colour. Said colour indicates that immediate intervention of a user is necessary in order to restore or preserve operability of the analysis system. In case the server computer determines that further tasks need to be performed not immediately but in the near future, said tasks are indicated by another colour, e.g. yellow. According to the embodiment depicted in Figure 2, the reagents subunit comprises enough reagent for performing the scheduled analyses but needs to be refilled soon. According to embodiments of the invention, the discrimination between tasks to be executed immediately and tasks to be executed in the near future can be specified by using threshold values, e.g. threshold values for a particular period of time until a consumable will be used up, a particular number of samples which can still be processed given a particular amount of consumables etc. For example, if the analysis system determines that a reagent bottle is filled only 20%, one or more tasks are automatically and dynamically specified for refilling said reagent bottle in the near future. If the bottle is empty or the filling level falls below a second threshold, one or more tasks are specified having assigned the highest urgency level and which need to be executed immediately.

Tasks which have to be executed immediately in or der to preserve or restore operability of the analysis system are referred to as tasks of highest priority. According to the depicted embodiment, the highest priority is indicated by red colour. Tasks which need to be executed soon but not immediately are indicated by a second colour, e.g. yellow. Said second colour represents a second urgency level. Tasks which can be executed by a user at any time are indicated by a third colour, e.g. grey or green, said third colour representing the lowest urgency level.

In the depicted embodiment, the one or more tasks to be executed immediately in respect to the consumables subunit are indicated by displaying a GUI element 217 representing the consumables subunit of "Cavallo" according to the colour indicating the highest urgency level. The corresponding one or more tasks 214 displayed in the in the tasks area 210 in the form of a link element comprise a coloured area 211 which is assigned the colour of the highest urgency level. The area 212 in the tasks area 210 and the "reagents" area 216 in the lab-device area 205 are assigned the colour representing the second urgency level (yellow).

According to the depicted embodiment, each task depicted in the tasks area 210 further comprises a text 224, 213 indicating the type of the aggregated tasks, e.g. "Supplies" or "Reagent" in case the aggregated task data objects relate to replenishing supplies or refilling reagents.

The tasks area 210 displays data aggregated on one or more tasks. GUI element 214, for example, is a link element indicating that the cuvette container of the lab-device Cavallo is empty and needs to be refilled. Refilling the cuvette container may relate to one single task or a multitude of tasks. For example, if the cuvette container comprises multiple different types of cuvettes which have to be replenished and have to be retrieved from different storage rooms, a multitude of task data objects indicating said tasks would be dynamically specified. GUI 200 provides the user with an aggregated view on one or more tasks to be executed by or in respect to Cavallo, thereby hiding details such as the type of the cuvettes to be replenished. As a result, the user is provided with a quickly comprehensible overview on the system state and urgent tasks. The link element 215 provides an aggregated view on 12 tasks which relate to 12 remaining glucose tests which could not be executed due to the unavailability of cuvettes. The aggregated view on tasks is highly advantageous, because in the context of a laboratory running large screens a list of tests to be performed on a set of samples may comprise several hundred or thousand items whose display in the form of a non-aggregated task list would disturb the user.

The group of squares 202 indicates the urgency level of the dynamically specified task data objects, the urgency level of a task data object representing the priority of the task indicated by said task data object. In case one or more tasks having assigned the highest urgency level are received by the server computer, the square at the left side turns into the colour of the highest urgency level, e.g. red. In case one or more tasks having assigned the second urgency level were determined, the central square turns into the colour of the second urgency level, e.g. yellow.

The lab-device status area 223 displays information on the current status of the lab-device displayed in lab-device area 205. Lab-device status area 223 in the depicted example indicates that all measurements were stopped and 12 biological samples still need to be processed by Cavallo.

Selectable GUI element "Routine" 207 is a tab element which represents a first category "Routine" and provides access to a corresponding drill-down graph. Likewise, selectable GUI elements "Lab" 208 and "Utility" 209 represent a second and third category, the second category corresponding to a second drill-down graph and the third category corresponding to a third drill-down graph. By selecting one of the selectable category GUI elements 207, 208, 209, a user can specify according to which drill-down graph a drill-down analysis is to be executed. Each task is indicated by a task data object dynamically received by the server computer.

The overview pane 201, herein also referred to as "home" pane, can display aggregated GUI elements representing aggregated task data objects also in case no user is logged into the analysis system. In case the analysis system comprises multiple lab-devices and tasks having assigned a high priority urgency level are assigned to more than one lab-device, the lab-device area 205 may also display multiple different lab-devices. In this case, the whole lab-device is colour-encoded according to the urgency level colour schema explained previously. The main function of the overview pane 201 is to inform a user irrespective of his role and assigned responsibilities on task which have to be immediately executed in order to keep the analysis system running.

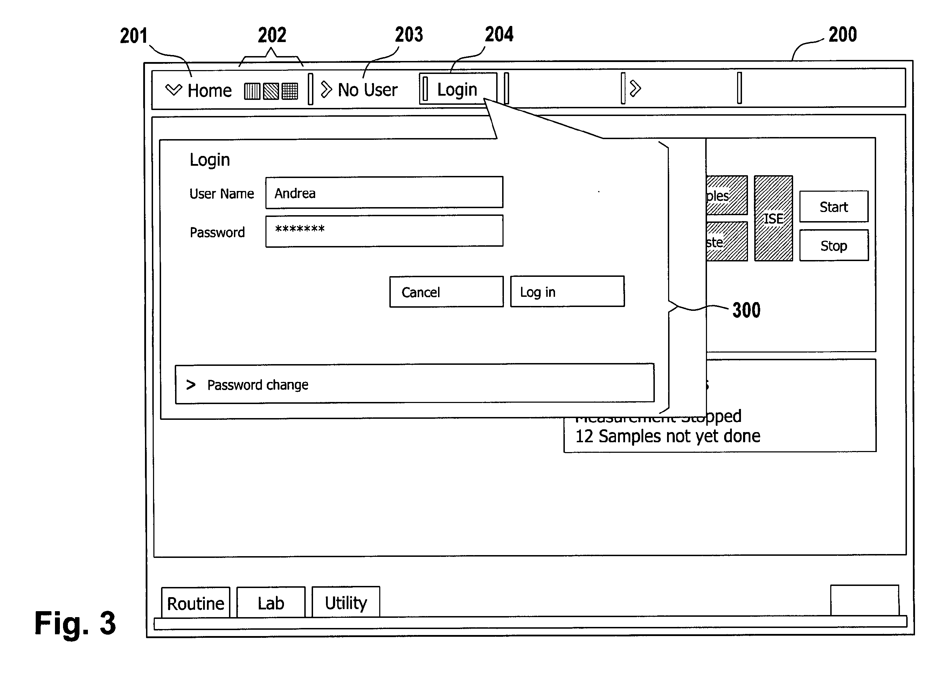

Figure 3 is a screenshot of a login dialog provided to a user e.g. upon selecting the login GUI element 204.

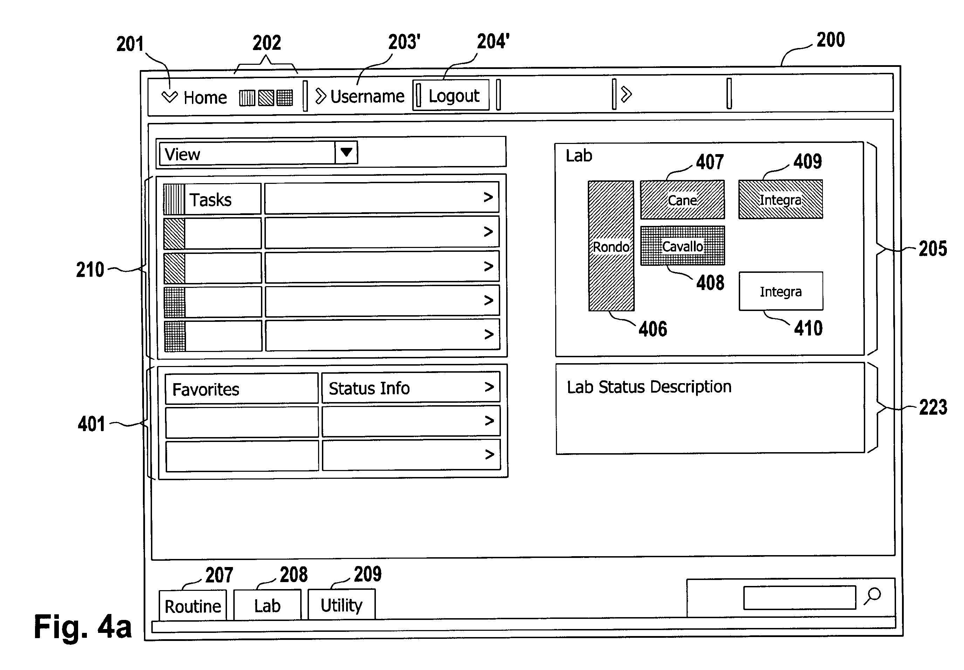

Figure 4a is a schematic drawing of an overview pane displayed when a user has successfully logged into the analysis system. The username of the logged-in user is displayed in GUI element 203'. Upon selection of GUI element 204', the user can log out again. Upon a successful login of a user, the overview pane 201 displayed to a logged-off user as described for figure 2 is complemented with user profile specific information, in particular user-specific tasks and favourites. The favourites pane 401 is a 2-column table comprising user-defined favourites in the first column and dynamically retrieved status information on each favourite item in the second column. The lab-device pane 205 displays a set of GUI elements 406-410 representing lab-devices "Rondo", "Cane", "Cavallo", "Integra 1" and "Integra 2" , said lab-devices being monitored by the server computer.

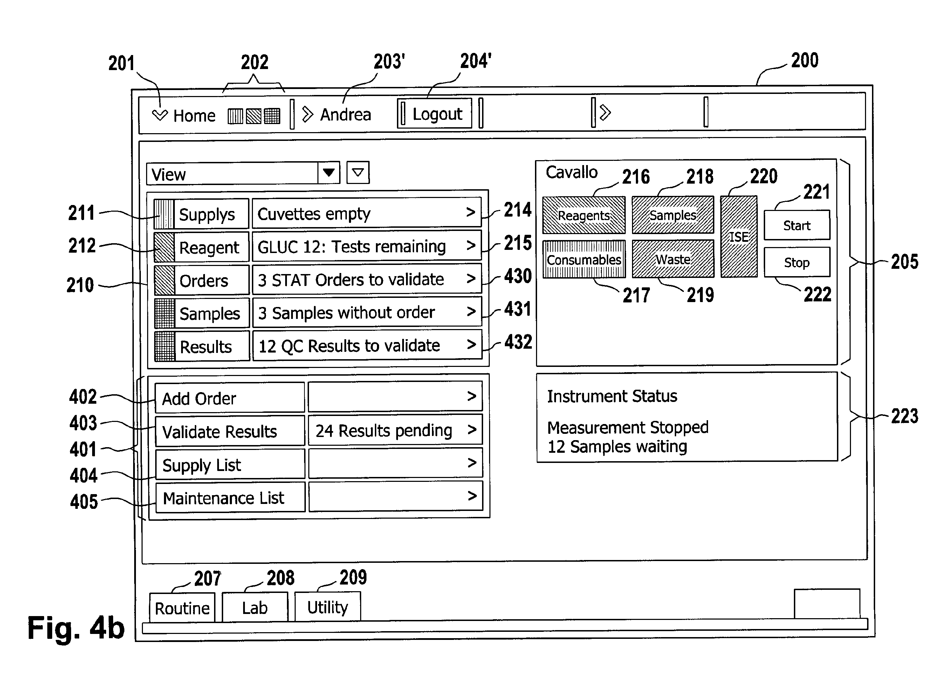

Figure 4b depicts the overview pane after its complementation with user-specific information. Tasks area 205 comprises GUI elements 430, 431 and 432, each providing an aggregated view on one or more user specific tasks. Said GUI elements are displayed in addition to the GUI elements 214, 215 providing an aggregated view on one or more tasks having assigned a high urgency level. The aggregated view provided to a logged-in user by the tasks area 205 urges a user to handle tasks having a high priority level before performing routine tasks.

The favourites pane 401 displays 4 favourites GUI elements 402-405 having been specified by the logged-in user. If available, status information for each favourite item is displayed. Each favourite GUI element represents a particular node in a drill-down graph, thereby representing also a particular aggregated view of task data objects. Each favourite GUI element 402-405 is a selectable GUI element allowing a user to navigate to aggregated view of task data objects represented by the selected favourite GUI element. Depending on the embodiment of the invention, favourite GUI elements may be specific for a user and/or a user role. For example, if it is the responsibility of a user to validate the results of a particular test, he may specify a favourite GUI element representing a node in a drill-down graph allowing the validation of the results generated in said test. As the specified favourite GUI element is displayed in the "home" pane 201, a user can save time by directly selecting favourite GUI element 403 instead of traversing a drill-down graph until a node and a corresponding aggregated view for evaluating analysis results is reached.

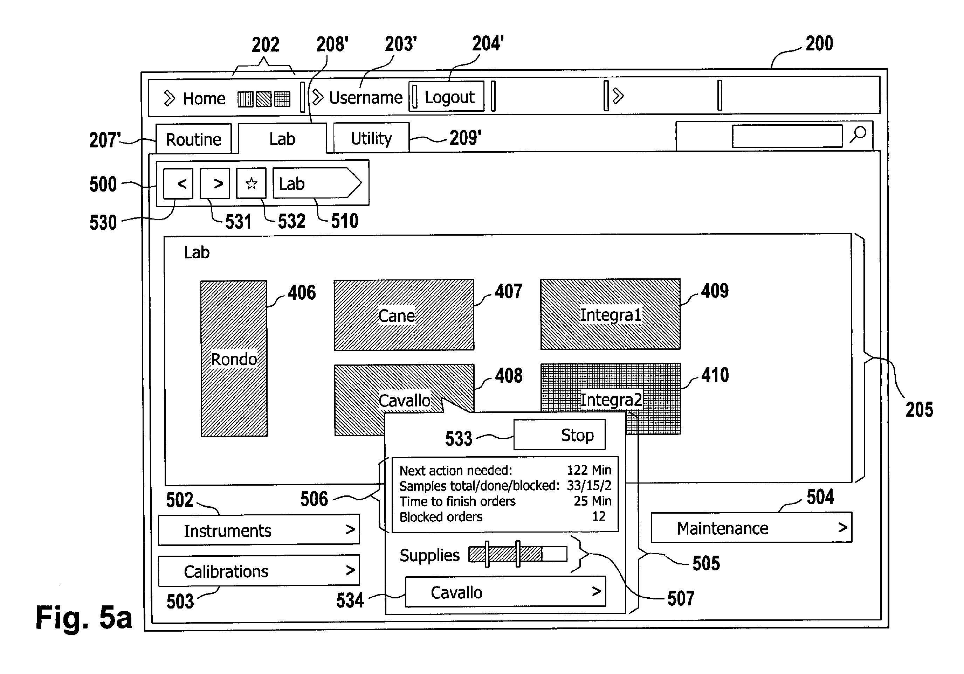

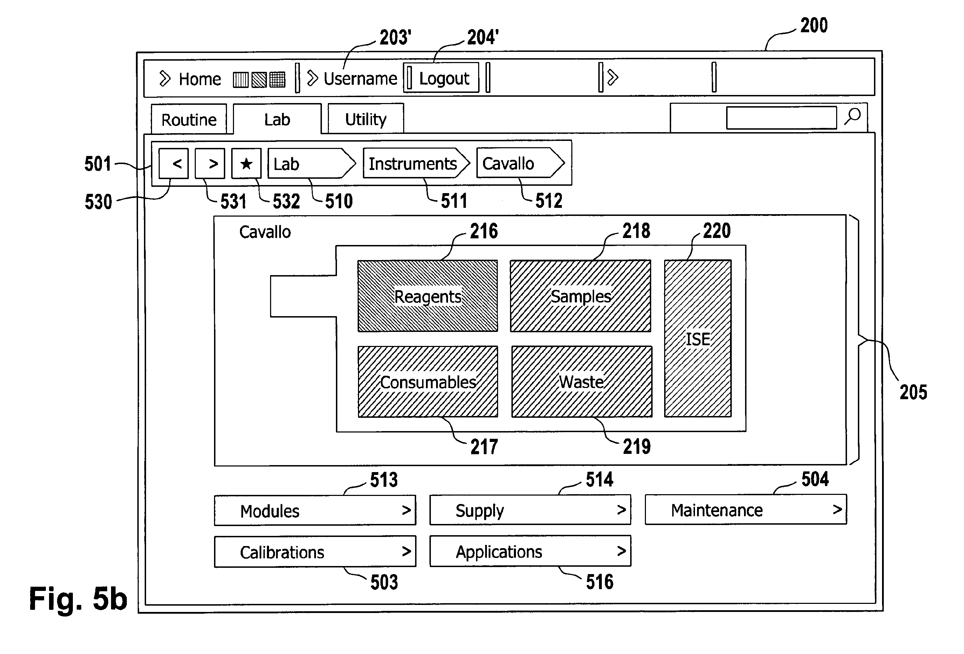

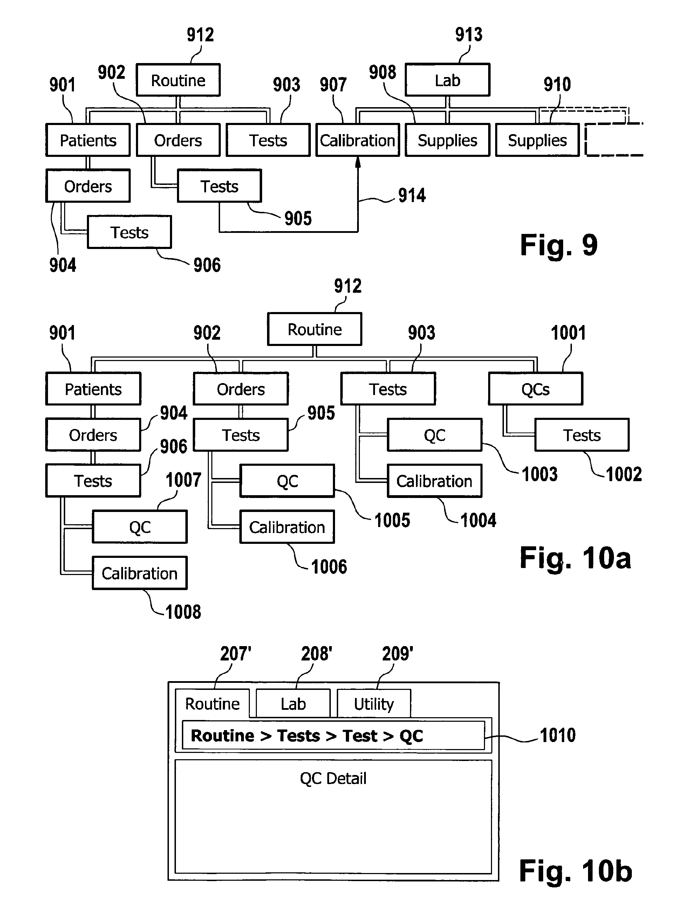

Figures 5a and 5b illustrate the execution o a drill-down analysis. Figure 5a depicts an overview on available lab-devices displayed upon selection of the 'Lab' category tab 208'. By selecting said 'Lab' category tab, the user also selects a particular drill-down graph which will be used for executing a drill-down analysis (see figure 9, drill-down graph "Lab"). The navigation path 500 indicates a user his current position within the drill-down graph. The navigation path 500 comprises a multitude of GUI elements: the selectable GUI element 530 allows a user to navigate "backward" in his chosen navigation path. The selectable GUI element 531 allows a user to navigate "forward", i.e. downwards in the selected drill-down graph provided the user already visited the selected nodes beforehand. The name of the currently selected node of the drill-down graph is displayed in a GUI element 510 at the right end of the navigation path 500. In order to allow the user to navigate to nodes the user has not yet visited, additional selectable GUI elements 502, 503, 504 are displayed which correspond to other nodes of the currently selected drill-down graph. As indicated by GUI element 510 in Figure 5a, the user has currently selected the "Lab" category drill-down graph and has selected the root node "Lab" as current node.

The selectable GUI element 532 allows a user to specify new favourites which are displayed after their specification in favourite area 401.

A user may control the operation of a particular lab-device, e.g. Cavallo, by selecting a GUI I element 408 representing said device with the right mouse button and selecting the "Stop" context menu element 533. The context menu 505 comprises status information 506 of the selected lab-device Cavallo. Upon selecting context menu item 534, a user can select a node in the 'Lab' drill-down graph representing the lab-device 'Cavallo' as grouping node, thereby triggering the execution of a drill-down analysis based on the selected grouping node 'Cavallo'. As a result, device-component-IDs of logical, functional and/or physical subunits of Cavallo are used as attribute in the grouping and aggregation step being executed during the triggered drill-down analysis. Accordingly, all task data objects being represented as direct or indirect successor nodes of the selected grouping node 'Cavallo' and having assigned the same device-component-ID or device-component-ID range are grouped into the same task data object group.

Depending on the embodiment, the execution of the drill-down analysis for a particular node, e.g. a particular lab-device, may likewise be triggered by other events, e.g. by clicking on the GUI element representing said lab-device with the left mouse button.

Figure 5b depicts an aggregated view on consumables, reagents and other items used by the lab-device 'Cavallo', the aggregated view being the result of a drill-down analysis having been executed for said lab-device by selecting the context menu item 534. The navigation path 501 comprises a set of navigation GUI elements 510, 511, 512 which specify a path in the drill-down graph starting from the root node "Lab" 913 represented by the tab 208' and ending in the currently selected grouping node "Cavallo" represented by GUI element 408. The lab-device pane 205 provides the logged-in user with an aggregated view on five groups of tasks which need to be executed on five logical, functional and/or physical components of Cavallo. In case a user selects a particular lab-device, e.g. Cavallo, as grouping node, all task data objects being grouped into the same group share at least a particular device-component-ID as attribute.

The selectable GUI elements 503, 513, 514, 516 displayed to the user correspond to other nodes of the currently selected drill-down graph. Upon selection by a user, the node represented by said selectable GUI element is used as current grouping node.

As a result of selecting the node representing the lab-device 'Cavallo' as grouping node in the 'Lab' drill-down graph, a drill-down analysis is executed and an aggregated view on task data objects having assigned Cavallo's device-ID is provided to the user as displayed in Figure 5b. The attribute used for grouping the direct and indirect successor nodes of the 'Cavallo' node is the device-component-ID. As a result of the drill-down analysis, 5 different groups of task data objects are created and 5 GUI elements providing an aggregated view on one or more task data objects are displayed: "Reagents", "Consumables", "Samples", "Waste", and "ISE". For each of said groups of task data objects, the data contained in the task data objects belonging to said group are aggregated to generate an aggregated data value. According to the depicted embodiment, the generated data value for each of said 5 groups is indicative of whether one or more task data objects representing tasks of the highest or second highest urgency level have been found. In case at least one task data object of a task data object group has assigned a high urgency level value, the colour of the GUI element representing said group turns into the colour representing said urgency level, e.g. red for the highest urgency level or yellow for the second highest urgency level.

For example, according to figure 5a, the GUI element 408 representing Cavallo and the GUI element 409 representing Integra1 are displayed in yellow colour. As a consequence, the GUI depicted in figure 5a indicates to a user that one or more tasks have to be executed on Cavallo and Integra1 in the near future (yellow represents the second highest urgency level). By selecting the node representing 'Cavallo' as grouping node, the user can command the server computer to provide him with a more detailed view on the tasks to be executed on Cavallo. As a result, a drill-down analysis is executed for the grouping node 'Cavallo' and an aggregated view on tasks to be executed in respect to five of Cavallo's logical, functional and/or physical subunits is provided to the user as shown in figure 5b. In Figure 5b, the reagents subunit GUI element 216 is displayed in yellow colour, thereby indicating that one or more tasks have to be executed which are related to the reagents compartment. By selecting said GUI element 216, the user can trigger the execution of a further drill-down analysis for the Reagents subunit of Cavallo. As a result, the user is provided with a more fine-grained aggregated view on the tasks which have to be performed by or in relation to the reagents subunit (not shown).

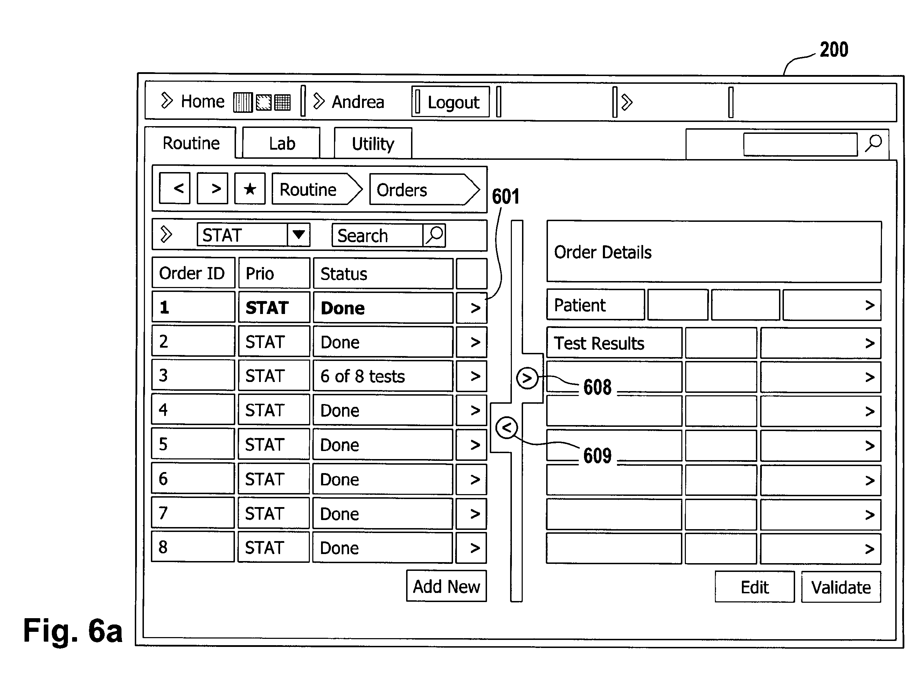

Figures 6a-6c illustrate the dynamic specification and aggregated display of tasks. The dynamic specification of new tasks in dependence on current system state allows a user working e.g. on some routine tasks to be informed immediately on a critical system state or a failure of a lab-device which requires immediate intervention by the user.

Figure 6a illustrates a graphical user interface resulting from selecting 'Routine' as current category and from selecting the node 902 'Orders' within the corresponding 'Routine' drill-down graph. On the left side of the pane a list of orders and their corresponding status is displayed. By selecting e.g. GUI element 601 corresponding to order ID 2, the details of the selected order are displayed in the 'order details' pane on the right side of the GUI and can be evaluated and verified by the user.

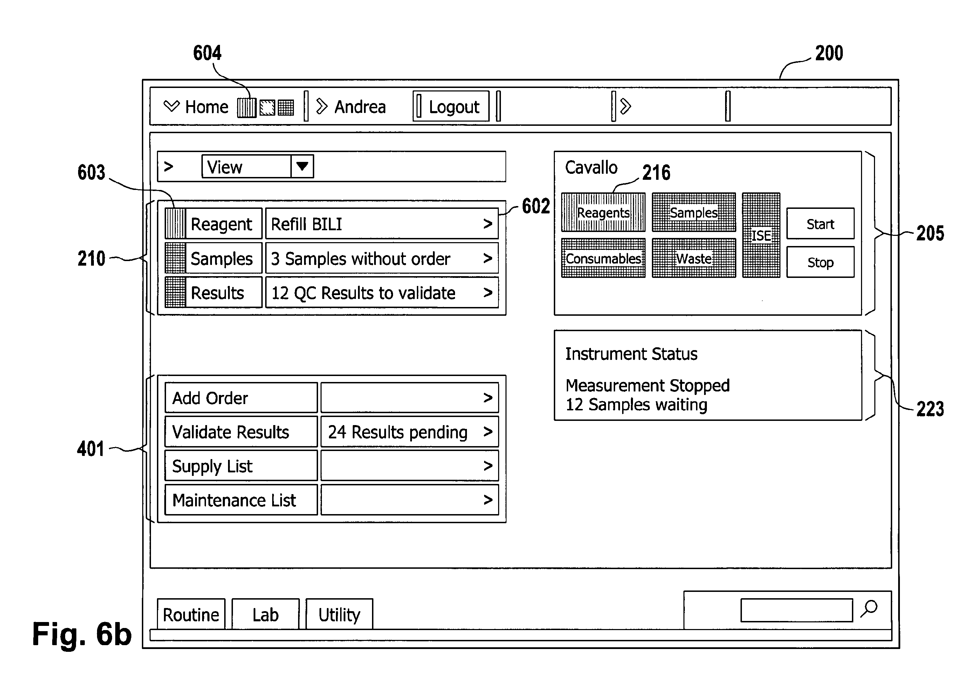

The server computer constantly receives status information of lab-devices belonging to the analysis system which are connected to the server computer. Such status information includes error codes, messages on used up reagents or consumables etc. In case a lab-device runs out of reagents, a new task data object is dynamically created, said task data object being indicative of the task to refill said reagent. Depending on the embodiment, different forms of warnings are then presented to the user, e.g. in the form of an acoustic signal and/or in the form of a refresh of the GUI, in particular of the group of squares 202. A user can navigate to the overview pane 201 which is depicted in figure 6b. The overview pane provides the user with an aggregated view of tasks having assigned a high urgency level, including the dynamically created task for refilling empty reagent bottles in Cavallo. The GUI element 602 in the tasks area represents the task of refilling a reagent required for a bilirubine analysis. By selecting GUI element 601, a user can trigger the display of data required for executing said tasks. As a result, the GUI elements contained in the lab-device area 205 are displayed. GUI element 216 in the lab-device area indicates that at least one reagent of the lab-device Cavallo needs to be refilled. GUI elements 603, 604 and 216 are displayed in red colour, thereby indicating that the reagent needs to be replaced immediately.

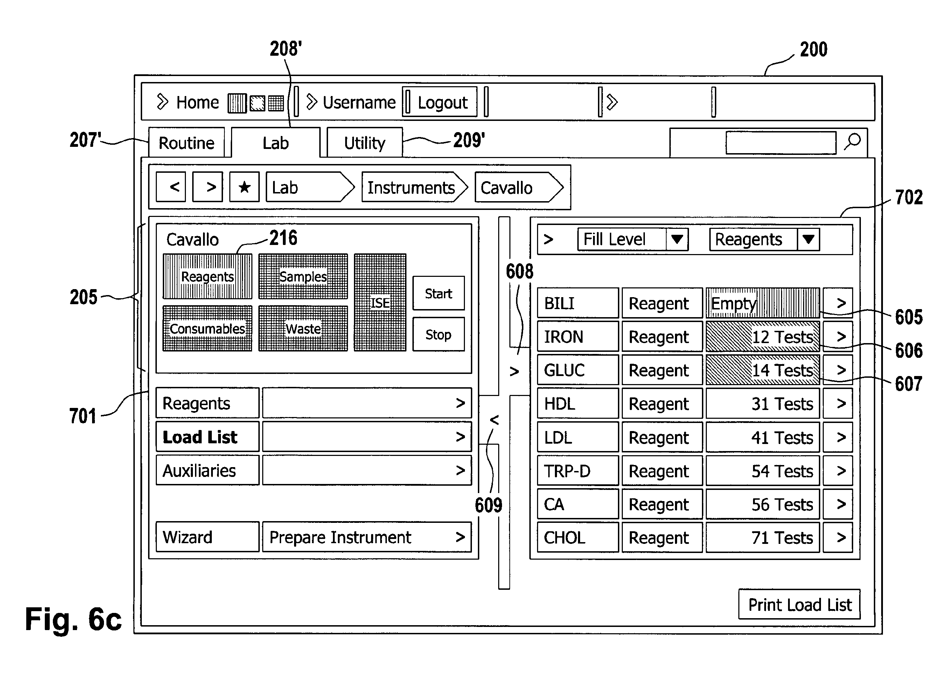

By selecting the GUI element 216 representing the reagents subunit of Cavallo, a user can trigger the display of further details as depicted in figure 6c. The content of the lab-device area 205, in figure 6b formerly displayed on the right half of the GUI, is depicted in the detailed view in figure 6c on the left half of the GUI. In pane 704, a list of reagents currently loaded in Cavallo is displayed. The empty reagent bottle for the BILI reagent is indicated to the user in red colour of GUI element 605. Other reagents which will need to be refilled in the near future are represented as yellow coloured GUI elements 606, 607. The list of reagents displayed in pane 702 is dynamically updated and can be printed.

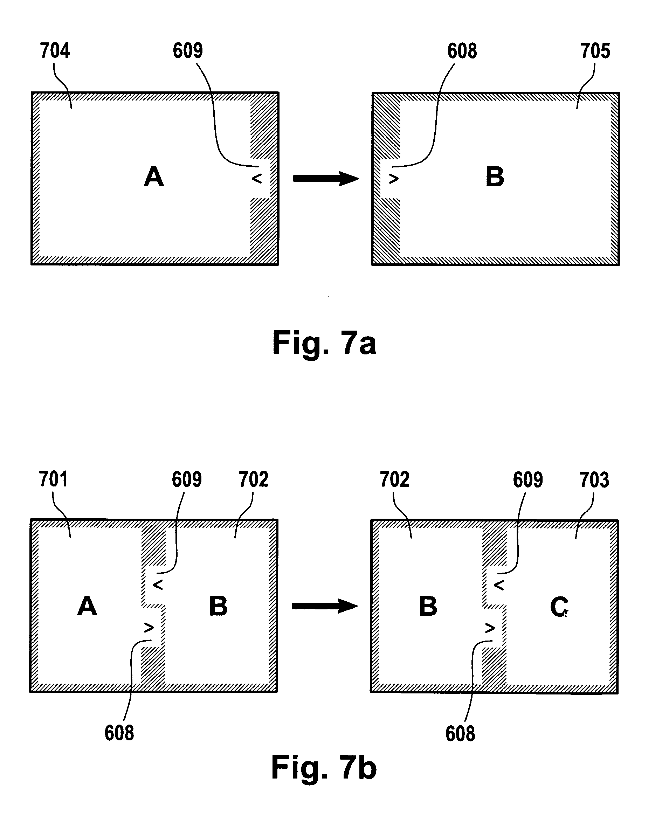

Figure 7a depicts an aggregated view generated as the result of a drill-down analysis to the user according to a first display mode. According to the first display mode, the GUI comprises a first pane 704 or a second pane 705, each of said panes being approximately of screen size. Each GUI element displayed in said first or second pane is provided according to a first GUI element version. Said version corresponds to a design specially adapted for the display of a GUI element on a small screen, in particular a screen of a mobile device. "Specially adapted" implies that the size and resolution of the displayed GUI elements allows the immediate recognition and comprehension by a user when presented to the user on a small screen. Each GUI element is a pointer to a node in the drill-down graph. At least one GUI element belonging to the first version of GUI elements is displayed in the first pane 704. Upon selection of said at least one first GUI element, the node being pointed at by the selected GUI element is used as grouping node and a drill-down analysis is executed for said grouping node. One or more second GUI elements generated as the result of said drill-down analysis are displayed in the second pane 705, the second pane being approximately of screen size. The second GUI elements are also provided according to said first GUI element version being specially adapted for displaying GUI elements on a small screen. The second pane replaces the first pane, thereby reducing the total amount of GUI elements to be displayed to the user at the same time on a small screen.

Figure 7b depicts an aggregated view according to a second display mode. According to the second display mode, the GUI comprises a first pane 701 and a second pane 702, each of said panes being approximately of half screen size. Each GUI element displayed in said first or second pane is provided according to a second GUI element version. Said version corresponds to a design specially adapted for the display of a GUI element on a medium sized screen, in particular a screen of a current computer system or touch screen of a lab-device. "Specially adapted" implies that the size and shape of the displayed GUI elements allows the immediate recognition and comprehension by a user when presented to the user on a medium-sized screen.

Each GUI element 214-220, 406-410, 430-432 is a pointer to a node in the drill-down graph (see e.g. figures 4a and 4b). At least one second version GUI element is displayed in the first pane 701, e.g. a link element. Upon selection of said at least one first GUI element, the node being pointed at by the selected GUI element is used as grouping node and a drill-down analysis is executed for said grouping node. One or more second GUI elements generated as the result of said drill-down analysis are displayed in the second pane 702, the second pane being approximately of half screen size. The second GUI elements are also provided according to said second GUI element version specially adapted for displaying GUI elements on a medium-sized screen. The first and the second pane are displayed to the user on the left and right side of the GUI at the same time. In case a user navigates further down in the drill-down graph, pane 702 displayed on the right side of the screen depicted in figure 6c and the left side of figure 7b will be moved to the left side of the screen, thereby replacing pane 701 while the right side of the screen displays the new pane 703.

Figure 6c corresponds to the second display mode according to which the first pane 701 and the second pane 702 are displayed simultaneously. The pagination GUI elements 608, 609 indicate that the content of the first and second pane can be changed by a user executing a drill-down analysis in a way similar to leaving through a book.

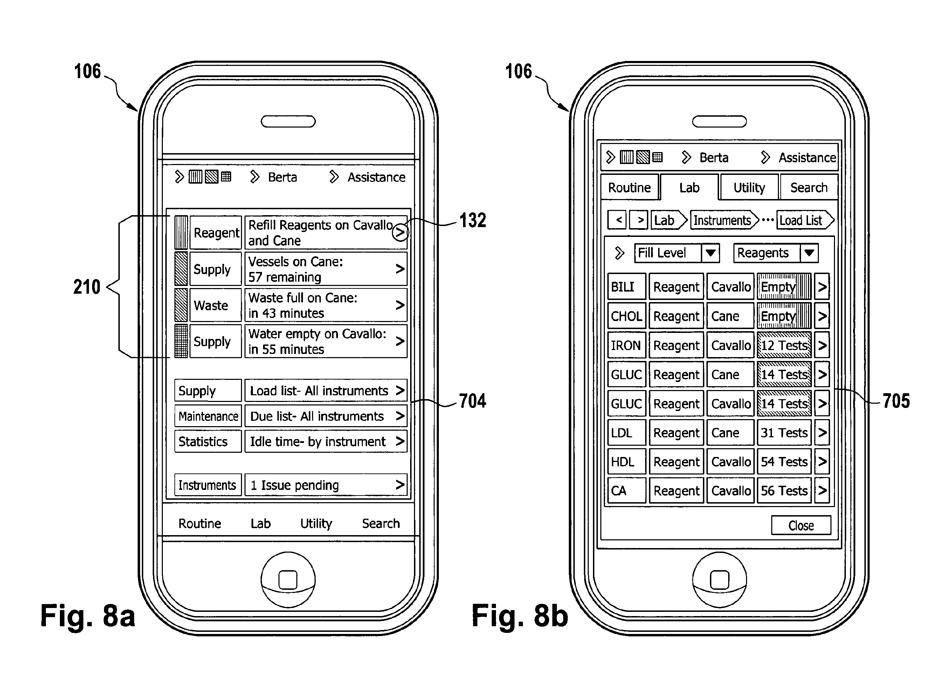

Figure 8a depicts a first pane 704 displayed on a screen of a mobile device 106 according to the first display mode. The first pane comprises the tasks pane and the favorites pane. Upon selection of the GUI element 132 representing one or more task data objects indicating the task of refilling reagents on Cavallo and Cane, a drill-down analysis is executed and the first pane 704 is replaced by the second pane 705 as displayed in figure 8b.

Figure 9 depicts parts of two different drill-down graphs corresponding to the categories 'Routine' and 'Lab', the drill-down graphs being connected via a link 914. Each box in figure 9 and 10a represents a node 901-913, 1001-1008 in a drill-down graph. Upon selection of tab 207', the drill-down graph having node 912 as root node is selected as drill-down graph. Accordingly, each of the tabs 207'-209' provide a user with different entry points for executing a drill-down analysis according to a particular graph topology, each topology corresponding to a different view of the data contained in the received task data objects added to the nodes of the drill-down graphs. The received task data object are added by the server computer to the nodes of each drill-down graph in dependence on the one or more attributes of each task data object (not shown). For example, a task data object indicating the task of executing a particular test on a particular lab-device by a particular user is only displayed to said logged-in user. By creating links between nodes of the same or of two different drill-down graphs, a business analyst can provide the end-user with the option to freely navigate between nodes of different drill-down hierarchies without generating circular or otherwise problematic drill-down analysis pathways.

Figure 10a is a detailed illustration of the drill-down graph corresponding to the category 'Routine'. As can be seen from figure 10a, multiple drill-down analyses can be executed starting from root node 912 and ending in a "Calibration" node 1004, 1006, 1008. According to a first pathway, the "Calibration" node 1008 is reached via nodes 912, 901, 904 and 906. According to alternative second pathway, the "Calibration" node 1006 can be reached via nodes 912, 902 and 905.

Figure 10b illustrates the relation between the navigation path 500 and 1010 and the corresponding path within the drill-down graph chosen for executing a drill-down analysis. The nodes 912 and 903 belonging to the third branch of the drill-down graph depicted in figure 10a correspond to the navigation path 1010 depicted in figure 10b after a user selected a particular quality control node "QC" for executing a quality check on measurement data received during the execution of a test.

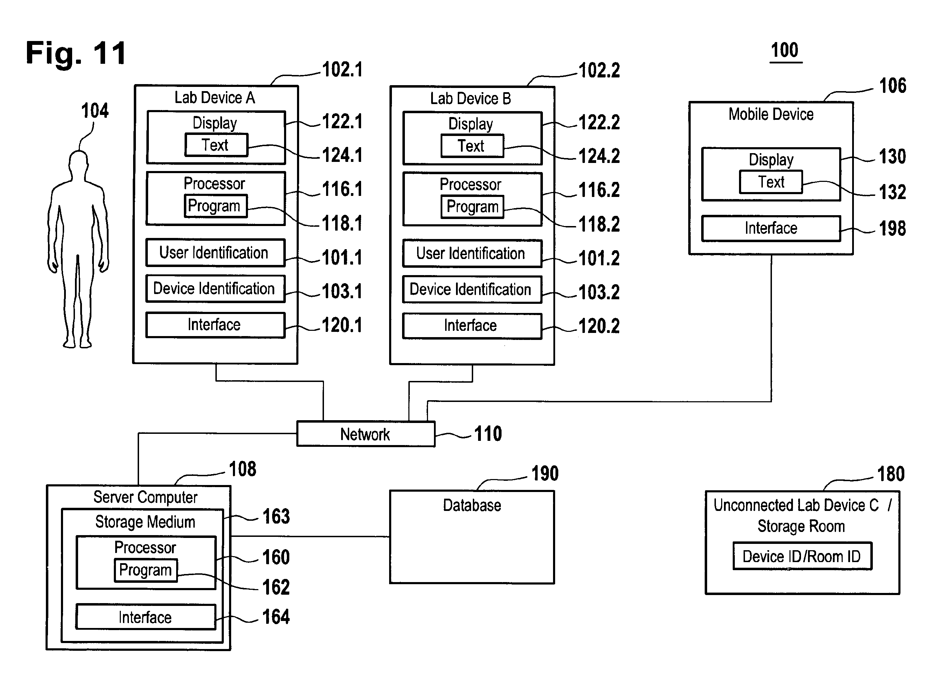

Figure 11 is a block diagram of an embodiment of an analysis system 100. The analysis system 100 comprises various lab-devices 102.1, 102.2 being coupled to a server computer 108 that controls operation of said lab-devices. According to the depicted embodiment, at least some of the lab-devices comprise a processor 116.1, 116.2 for executing computer-implemented instructions 118.1, 118.2 which allow the specification of task data objects and/or are required for exchanging data with the server computer. Depending on the implementation at least some of the lab-devices are coupled to the server computer 108 via the network 110; In the embodiment considered here the analysis system 100 has a connected lab-device A and a connected lab-device B. The connected lab-device A has at least one processor 116.1 for execution of program instructions 118.1. The connected lab-devices have a network interface 120.1, 120.2 for coupling the connected lab-device A, B to the network 110, in particular in order to enable communication with a server computer 108. The unconnected lab-device 180 can be, for example, a refrigerator, a storage room for storing reagents or samples and the like. Although said unconnected lab-device or room is not capable of directly exchanging data with the server computer, the device-ID or room-ID of said unconnected storage room or unconnected lab-device can be assigned to a task data object and used in the drill-down analysis.

The network 110 can be a cable based network, such as a local area network, an Ethernet or the like, or a wireless network, such as a WIFI, GSM, UMTS or other wireless digital network. In particular, the network 110 can implement the Internet protocol (IP), in particular mobile IP. For example, the network 110 comprises a combination of cable-based and wireless networks.

The connected lab-device A has a display, such as a computer monitor 122.1 for displaying a screen image 124.1 that may include a textual output.

Further, the connected lab-device A has a user identification component 101.1 and a device identification component 103.1.

The user identification component 101.1 serves for identification of a human user 104, such as a laboratory assistant. The user identification component 101.1 can be operable for the user's 104 entry of his or her user name and password. Alternatively or in addition the user identification component 101.1 can implement an RFID method for determining the user's 104 identity by means of an RFID chip card of that user. As an alternative or in addition the user identification component 101.1 can be operable to perform the user's 104 identification using a biometric method, such as fingerprint identification. In this instance the user identification component 101.1 comprises a sensor for sensing the user's 104 respective biometric features, such as a fingerprint sensor.

The device identification component 103.1 can be a protected storage area of the connected lab-device A in which a unique device identifier for identification of the device is stored. This device identifier can be a serial number, such as a globally unique identifier (GUID) that is assigned to the connected lab-device A during its production or it can be an identifier that is unique within the analysis system and that is assigned to the connected lab-device A by an administrator of the analysis system. Alternatively the device ID is not permanently stored by the device identification component 103.1 but the device ID is computed by the device identification component 103.1 each time an identification of the connected lab-device A is required. The computation of the device ID can be executed by the device identification component 103.1 using a predefined algorithm, such as a cryptographic algorithm.

The connected lab-device B of the analysis system 100 has a design that is analogous to the connected lab-device A. Thus, the connected lab-device B has a user identification component 101.2, a device identification component 103.2, a processor 116.2 for execution of program instructions 118.2, a monitor 122.2 for displaying a screen image 124.2 and a metric interface 120.2 corresponding to the respective components of the connected lab-device A. The analysis system 100 in addition may comprise one or more unconnected lab-devices, such as unconnected lab-device C 180. For example, the unconnected lab-device C is a refrigerator for storing reagents that are required for operating the various analyzers of the analysis system 100.

The analysis system 100 can have a plurality of additional connected lab-devices not shown in figure 1 that are also coupled to the server computer 108 over the network 110.

The server computer 108 has a processor 160 for execution of program instructions 162, and a network interface 164 for coupling the server computer 108 to the network 110. The instructions 162 are stored on a computer-readable, non-transitory storage medium, e.g. a magneto-electric data storage, a Flash memory or the like. The program instructions 162 comprise a program module for receiving, creating, modifying and/or deleting task data objects stored in a storage component 190 that is coupled to the server computer 108. The storage component 190 can form an integral part of the server computer 108 or it can be a separate component such as a database server that is either directly coupled to the server computer 108 or via the network 110.

In operation, the user 104 logs into the analysis system 100, e.g. via connected lab-device A. According to other embodiments, the user 104 may likewise log into the analysis system via his mobile device 106 which can communicate with the server computer 108. By operation of the user identification component 101.1 the user 104 is identified, such as by an entry of a user identifier.

The user's 104 identifier and the device identifier of the connected lab-device A provided by the device identifier component 103.1 are sent from the interface 120.1 of the connected lab-device A to the server computer 108 via the network 110. This invokes the execution of the program instructions 162 which selects and processes task data objects having assigned at least the identifier of the identified user and the device identifier received from the connected lab-device A. For example, the program 162 queries database 190 in which the task data objects are stored using the user identifier and/or the device identifier as a search term in order to retrieve task data objects that are assigned to that user.

By execution of the program 162 the processor 160 generates a signal that provides a user with a graphical user interface as displayed e.g. in figure 4b. This signal provides an aggregated view on one or more task data objects and is sent from the server computer 108 via the network 110 to the connected lab-device identified by the device identifier, i.e. connected lab-device A.

In response to receipt of this signal by the connected lab-device A, execution of the program instructions 118.1 is invoked such that the screen image 124.1 is displayed on the monitor 122.1 including the textual output being descriptive of one or more tasks indicated by the one or more task data objects contained in the signal. As a consequence the user 104 is informed regarding the one or more tasks to be executed by that user.

The signal may likewise be sent to the mobile device 106 of the user 104, whereby the GUI elements displayed to the user in this case belong to the first version of GUI elements. The mobile device 106 has an integrated display 130 for displaying a screen image 132 that may contain a textual or other visual output. Further, the mobile device 106 has a network interface 198 for coupling to the network 110.

In operation, the user 104 selects one of the connected lab-devices A or B, such that the server computer 108 receives a user identifier and a device identifier. Alternatively the user may select the mobile device 106 such that a user identifier and a mobile device identifier are received by the server computer 108 instead. The user identifier and the device identifier are used by the program 162 for determination of one or more task data object to be executed by the user 104 by means of the database 190.

The signal sent to the mobile device 106 of the user 104 or to any of the lab-devices can comprise information regarding the device-id or room-ID where one or more tasks have to be executed. For example, the signal can comprise a set of task data objects indicting that several reagents need to be refilled and that fresh reagent bottles can be retrieved from the unconnected lab-device C 180. Each of said task data objects comprises information on the room number where said lab-device C, in this case a refrigerator, can be found. In case multiple tasks have to be executed by the user in respect to said unconnected lab-device, the user is provided with an aggregated view on said multitude of tasks.

Upon receipt of this signal by the mobile device 106 the user 104 can walk to the unconnected lab-device C for execution of the determined one or more tasks. After completion of the execution of the determined step the user 104 may use the mobile device 106 in order to acknowledge completion of the execution which is signalled from the mobile device 106 back to the server computer 108 in order to mark the said tasks as completed in the database 190.

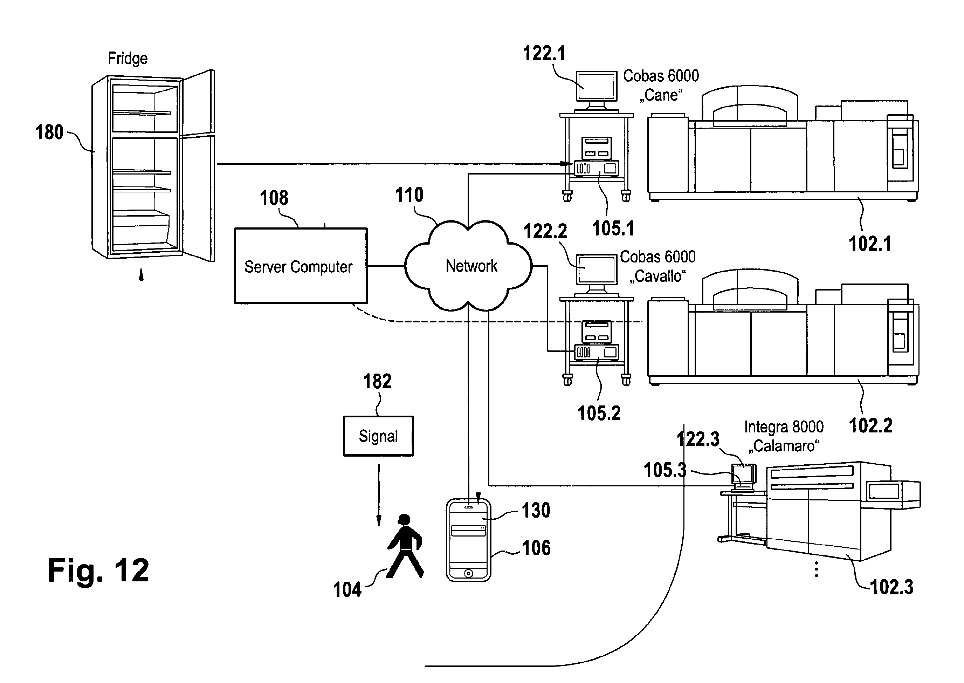

Figure 12 is a block diagram of a further embodiment of an analysis system of the invention where the requirement for refilling a reagent is signalled to a user. The depicted analysis system comprises an analyser 102.1 of the cobas 6000 type having the device ID 'Cane', analyser 102.2 of the cobas 6000 type having the device ID 'Cavallo' and analyser 102.3 of the Integra 800 type having the device ID 'Calamaro'. The analyser 102.1 and its analyser control computer 105.1 constitute connected lab-device A and the analyser 102.2 and its analyser control computer 105.2 constitute connected lab-device B. The analysis system further comprises the unconnected lab-device 180 which is a refrigerator for storing a stock of reagents.

In operation, one of the analyzers, such as the analyser 102.1, requires refilling of a reagent. For example, this is sensed by means of a sensor that is coupled to the analyser control computer 105.1. When the charging level of the reagent of the analyser 102.1 is below a predefined threshold level, the analyser control computer 105.1 generates a signal 182 that is sent to the mobile device 106, depending on the embodiment of the invention, either directly or through the intermediary of the server computer 108. According to embodiments wherein the signal 182 is sent directly from the analyser control computer 105.1 to the mobile device, the analyzer control computer is operable, in addition to the server computer, to execute the grouping and aggregating of task data objects as described for the server computer 108 or is operable to receive the results of grouping and aggregating the received task data objects from the server computer 108. According to further embodiments the server computer 108 receives task data objects, executes a grouping and aggregation step on the received task data objects and sends a signal 182 directly to the mobile device of a user. In one embodiment the analyser control computer 105.1 sends the signal 182 to the server computer 108 which then selects the mobile user device 106 for forwarding the signal 182.

The signal 182 comprises data aggregated on task data objects which have each assigned the device-id of an identified lab-device, said signal 182 being sent to the identified lab-device and/or to a mobile device of a user. In case the signal 182 comprising the aggregated data is sent from an analyser control computer 105.1

### List of reference numerals

- 100: analysis system
- 101: user identification component
- 102: lab-device
- 103: device-identification component
- 104: user
- 105: analyzer control computer
- 106: mobile user device
- 108: server computer
- 110: network
- 116: processor
- 118: program instructions
- 120: network interface
- 122: monitor
- 124: screen image
- 130: display
- 132: GUI element
- 160: processor
- 162: program instructions
- 163: storage medium
- 164: interface
- 180: unconnected lab-device/storage room
- 182: signal
- 190: database
- 198: network interface
- 201-205: step
- 200: graphical user interface
- 201: overview pane
- 202: group of squares
- 203: GUI element, user logged-off
- 203': GUI element, user logged-in
- 204: GUI element, user logged-off
- 204': GUI element, user logged-in
- 205: lab-device area
- 207: 'Routine' tab
- 208: 'Lab' tab
- 209: 'Utility' tab
- 210: tasks area
- 211: colored area and
- 212: coloured area
- 213: text indicating task type
- 214: link element "cuvettes empty" indicating one or more tasks
- 215: link element "glucose tests remaining" indicating 12 remaining tasks
- 216: GUI element for lab-device subunit "reagents"
- 217: GUI element for lab-device subunit "consumables"
- 218: GUI element for lab-device subunit "samples"
- 219: GUI element for lab-device subunit "waste"
- 220: GUI element for lab-device subunit "ISE"
- 221: selectable GUI element "start"
- 222: selectable GUI element "stop"
- 223: lab-device status area
- 224: text indicating task type
- 300: login dialog window
- 401: favorites area
- 402: favourites GUI element
- 403: favourites GUI element
- 404: favourites GUI element
- 405: favourites GUI element
- 406: GUI element for lab-device "Rondo"
- 407: GUI element for lab-device "Cane"
- 408: GUI element for lab-device "Cavallo"
- 409: GUI element for lab-device "Integra1"
- 410: GUI element for lab-device "Integra2"
- 430: link element "STAT orders to validate" indicating one or more tasks
- 431: link element "samples without order" indicating three tasks
- 432: link element "QC results to validate" indicating 12 tasks
- 500: navigation path
- 501: navigation path
- 502: selectable GUI element "instruments"
- 503: selectable GUI element "calibrations"
- 504: selectable GUI element "maintenance"
- 505: context menu
- 506: status information on selected device
- 507: filling level of supplies
- 510: navigation GUI element
- 511: navigation GUI element "instruments"
- 512: navigation GUI element "Cavallo"
- 513: selectable GUI element "modules"
- 514: selectively GUI element "supply"
- 516: selectively GUI element "applications"
- 530: selectable GUI element "back"
- 531: selectable GUI element "forward"
- 532: selectively GUI element "create favourite"
- 532: selectable GUI element "stop device"
- 534: context menu item
- 601: selectable GUI element
- 602: link element "refill BILI" indicating one or more tasks
- 603: coloured area
- 605: status information "empty reagent"
- 606: status information "12 tests remaining"
- 607: status information "14 tests remaining"
- 608: pagination GUI element
- 609: pagination GUI element
- 701: first pane
- 702: second pane
- 703: third pane
- 704: first pane
- 705: second pane
- 912: root node of the drill-down graph of the "routine" category
- 913: root node of the drill-down graph of the "lab" category
- 901-906: nodes
- 907-910: nodes
- 1001-1008: nodes
- 1010: navigation path

## Claims

1. An analysis system for analyzing biological samples comprising:
- at least one lab-device (102.1, 102.2),
- a server computer (108) having a server interface component (164) for receiving (201) task data objects, each task data object comprising at least one attribute, wherein at least some of the received task data objects are indicative of a laboratory procedure to be performed on at least one biological sample using the at least one lab-device (102.1, 102.2),
the server computer (108) comprising:
• a processing component (160) for:
- grouping (202) the received task data objects into task data object groups, wherein all task data objects belonging to the same task data object group share the same attribute value or attribute value range of the at least one attribute,
- for at least some of the task data object groups, aggregating (203) all task data objects contained in a task data object group to provide aggregated task data object groups, each aggregated task data object group comprising an aggregated data value, said aggregated data value being calculated from all task data objects being contained in said aggregated task data object group, and
- specifying (204) a GUI element (214-220) for each of the aggregated task data object groups, whereby the aggregated data value calculated for said aggregated task data object group is used for specifying said GUI element, said specified GUI element being representative of said aggregated task data object group, and
• a graphical user interface (122.1, 122.2, 130, 200) for
- displaying (205) the specified GUI elements for providing an aggregated view.

2. The analysis system according to claim 1,
• wherein a device-ID is assigned to at least some of the received task data objects, each device-ID being indicative of one of the at least one lab-devices,
• wherein the at least one lab-device comprises
o a device identification component (103.1, 103.2) for identifying the lab-device (102.1, 102.2) at the server computer (108) via a device-ID,
• wherein the server computer comprises
o a service interface component (164), wherein the server interface component is operable to send a signal (182) to the identified lab-device via the service interface component (164), the signal comprising data aggregated on the received task data objects to which is assigned the device-ID of the identified lab-device.

3. A method being implemented by an analysis system (100), the analysis system being used for processing biological samples, the method comprising the steps of:
A) receiving (201) task data objects, each task data object comprising at least one attribute, wherein at least some of the task data objects are indicative of a laboratory procedure to be performed on at least one biological sample using at least one lab-device (102.1, 102.2), the at least one lab-device belonging to the analysis system (100),
B) grouping (202) the received task data objects into task data object groups, wherein all task data objects belonging to the same task data object group share the same attribute value or attribute value range of the at least one attribute,
C) for at least some of the task data object groups, aggregating (203) all task data objects contained in a task data object group to provide aggregated task data object groups, each aggregated task data object group comprising an aggregated data value, said aggregated data value being calculated from all task data objects being contained in said aggregated task data object group,
D) specifying (204) a GUI element (214-220) for each of the aggregated task data object groups, whereby the aggregated data value calculated for said aggregated task data object group is used for specifying said GUI element, said specified GUI element being representative of said aggregated task data object group, and
E) displaying (205) the specified GUI elements on a graphical user interface (122.1, 122.2, 130, 200) for providing an aggregated view.

4. The method according to claim 3, the method further comprising the steps of:
- representing each received task data object as a node of a hierarchical drill-down graph, the hierarchical drill-down graph comprising at least two hierarchical levels,
wherein the grouping step B comprises:
- determining one grouping node of the hierarchical drill-down graph, the grouping node being a starting point for executing a drill-down analysis, the drill-down analysis being a data aggregation operation executed on the grouping node and all direct and indirect successor nodes of the grouping node,
- wherein all task data objects being grouped into the same task data object group in the grouping step B share the same attribute value or attribute value range of the at least one attribute.

5. The method according to claim 4, further comprising the steps of:
- displaying at least one navigation GUI element (510-512), wherein the at least one displayed navigation GUI element is a selectable pointer to a node of the drill-down graph,
- upon selection of said at least one navigation GUI element by a user, using the node being pointed at by the selected navigation GUI element as grouping node, and
- triggering the execution of steps B-E, thereby providing an aggregated view on data contained in task data objects which are represented as direct or indirect successor nodes of the used grouping node.

6. The method according to anyone of claims 4-5, further comprising the steps of:
- displaying two or more navigation GUI elements (510-512) on the GUI,
- wherein the two or more navigation GUI elements constitute a navigation path (500, 501, 1010), the navigation GUI elements of said navigation path connecting nodes having already been used as grouping node in a drill-down analysis, the navigation path allowing the user to navigate backward and forward between nodes of the drill-down graph by selecting navigation GUI elements pointing to said nodes, and
- wherein the last navigation GUI element (510, 512) in said navigation path (500, 501) is a pointer to a node having been used as grouping node for executing a drill-down analysis, and wherein the GUI elements having been specified in said drill-down analysis are currently displayed.

7. The method according to anyone of claims 4-6, further comprising the steps of:
- storing, for each drill-down analysis, at least the aggregated data values in a drill-down history, the drill-down history being stored in a working memory,
- selecting a navigation GUI element (510-512) by a user, the user thereby selecting a grouping node, the grouping node corresponding to a previously executed drill-down analysis,
- loading the stored aggregated data values aggregated in said previously executed drill-down analysis, thereby making a re-execution of steps B-E unnecessary.

8. The method according to anyone of claims 4-7, further comprising the steps of:
- specifying one or more categories, wherein each category is selected from a group comprising
- biological sample related data,
- lab-device related data, and
- configuration data, the configuration data comprising
■ configuration information of one or more tests performed on the at least one sample, and/or
■ configuration information of the at least one lab-device,
- representing each of the one or more categories as a selectable category GUI element (207, 207',208, 208', 209, 209'), in particular as tab,
- creating, for each category, a corresponding drill-down graph,
- adding each of the one or more nodes representing a received task data object to each of the created one or more drill-down graphs,
- upon selection of one of the one or more category GUI elements by a user, selecting the drill-down graph corresponding to the selected category GUI element, and
- executing steps B-E, wherein steps B and C are executed according to the graph topology of the selected drill-down graph.

9. The method according to claim 8, further comprising the steps of:
- creating at least one link, the at least one link connecting a first node with a second node,
- wherein the first node belongs to a first drill-down graph, the first drill-down graph corresponding to a first category,
- wherein the second node belongs to the first drill-down graph or to a second drill-down graph, the second drill-down graph corresponding to a second category.

10. The method according to anyone of claims 3-9, wherein each task data object is assigned an urgency level attribute, further comprising the step of determining that no user is currently logged-in,
wherein during the execution of step C each aggregated task data object group is assigned an aggregated urgency level value, the assigned urgency level value being calculated as the maximum urgency level attribute value assigned to any of the task data objects within said aggregated task data object group,
wherein in step E selectively GUI elements of those groups of task data objects are displayed whose aggregated urgency level value is above a threshold.

11. The method according to anyone of claims 4-10, further comprising:
- providing each of the GUI elements according to a first GUI element version, a GUI element version corresponding to a particular graphical design, the first GUI element version being adapted to be displayed on a small screen, in particular a screen of a mobile user device (106), wherein each GUI element is a pointer to a node (901-913, 915-1008) in the drill-down graph,
- displaying first GUI elements in a first pane (704), the first pane approximately being of screen size,
- upon selection of one of said first GUI elements, using the node being pointed at by the selected GUI element as grouping node and executing a drill-down analysis by executing steps B-E,
wherein the GUI elements displayed in step E are second GUI elements, said second GUI elements being displayed in a second pane (705), the second pane approximately being of screen size, the second pane replacing the first pane.

12. The method according to anyone of claims 4-11, further comprising:
- providing each of the GUI elements according to a second GUI element version, a GUI element version corresponding to a particular graphical design, the second GUI element version being adapted to be displayed on a medium-sized screen, in particular a screen of a computer system or a screen of a lab-device, wherein each GUI element is a pointer to a node (901-913, 915-1008) in the drill-down graph,
- displaying first GUI elements in a first pane (701), the first pane approximately being of half screen size,
- upon selection of one of said first GUI elements, using the node being pointed at by the selected GUI element as grouping node and executing a drill-down analysis by executing steps B-E,
wherein the GUI elements displayed in step E are second GUI elements, said second GUI elements being displayed in a second pane (702), the second pane approximately being of half screen size, the first pane and the second pane being displayed at the same time.

13. The method according to anyone of claims 3-12, wherein each task data object group generated in step B and aggregated in step C is graphically represented as link element, each link element providing a user access to program instructions for executing the tasks indicated by the task data objects being represented by said link element.

14. The method according to anyone of claims 3-13, the method further comprising:
- monitoring the status of the at least one lab-device to receive status information of said at least one lab-device,
- dynamically creating, modifying and/or deleting at least some of the received task data objects in dependence on the received status information, and
- triggering a re-execution of the steps B-E,
wherein as a result of said re-execution a dynamically updated, aggregated view of tasks to be executed is provided.

15. A computer-readable, non-transitory storage medium (163) having stored therein instructions (162) that when executed by a processor (160) cause the processor to perform a method according to anyone of claims 3-14.
